# EUROPEAN PATENT SPECIFICATION

(11) **EP 2 032 721 B1**
(45) Date of publication and mention of the grant of the patent: **29.03.2017**
(21) Application number: 07748704.9
(22) Date of filing: 04.06.2007
(51) Int. Cl.: C12Q 1/68, C12N 15/10

(54) **NUCLEIC ACID CONCATENATION**
NUKLEINSÄUREKONKATENATION
CONCATENATION D'ACIDE NUCLEIQUE

(30) Priority: 09.06.2006 US 449872
(43) Date of publication of application: 11.03.2009
(73) Proprietor: Agency for Science, Technology and Research, Singapore 138632 (SG)
(72) Inventor: RUAN, Yijun, Singapore 138672 (SG); NG, Patrick Wei Pern, Singapore 119174 (SG); FULLWOOD, Melissa Jane, Singapore 138672 (SG); LEE, Yen Ling, Singapore 542117 (SG)
(74) Representative: Arends, William Gerrit
(86) International application number: PCT/SG2007/000159
(87) International publication number: WO 2007/142608

(56) References cited:
- EP-A2- 0 761 822
- WO-A1-2004/024953
- WO-A1-2006/003721
- WO-A1-2006/003721
- WO-A2-2005/042781
- US-A1- 2004 146 866
- US-A1- 2005 059 022
- WEI C-L ET AL: "5' Long serial analysis of gene expression (LongSAGE) and 3' LongSAGE for transcriptome characterization and genome annotation" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0403514101, vol. 101, no. 32, 10 August 2004 (2004-08-10), pages 11701-11706, XP002380745 ISSN: 0027-8424
- NG P ET AL: "GENE IDENTIFOCATION SIGNATURE (GIS) ANALYSIS FOR TRANSCRIPTOME CHARACTERIZATION AND GENOME ANNOTATION" NATURE METHODS, NATURE PUBLISHING GROUP, GB LNKD- DOI:10.1038/NMETH733, vol. 2, no. 2, 1 February 2005 (2005-02-01), pages 105-111, XP009058027 ISSN: 1548-7091
- DUNN JOHN J ET AL: "Genomic signature tags (GSTs): A system for profiling genomic DNA" GENOME RESEARCH, COLD SPRING HARBOR LABORATORY PRESS, WOODBURY, NY, US, vol. 12, no. 11, 1 November 2002 (2002-11-01), pages 1756-1765, XP002304617 ISSN: 1088-9051
- CHUM W.W.Y. ET AL.: 'Modification of LongSAGE for obtaining and cloning long concatemers' BIOTECHNIQUES vol. 39, no. 5, 2005, pages 637 - 640, XP008090548
- ZHANG Z. ET AL.: 'Mapping of transcription start sites in Saccharomyces cerevisiae using 5' SAGE' NUCLEIC ACIDS RESEARCH vol. 33, no. 9, 2005, pages 2838 - 2851, XP008090942

## Description

### Field of the Invention

The present invention generally relates to the field of nucleic acids. Specifically, the present invention relates to concatenation of nucleic acids.

### Background of the Invention

One of the most important goals of the human genome project is to provide complete lists of genes for the genomes of human and model organisms. Complete genome annotation of genes relies on comprehensive transcriptome analysis by experimental and computational approaches. *Ab initio* predictions of genes must be validated by experimental data. However; due to the complexity and immense volume of transcripts expressed in the various developmental stages of an organism's life cycle, complete sequencing analysis of all different transcriptomes still remains unrealistic.

In the past decade this problem has been overcome with a cDNA tagging method in which partial sequences that represent full transcripts are obtained. This strategy has been widely used for determining genes and characterizing transcriptomes.

In the expressed sequence tag (EST) approach, cDNA clones are sequenced from 5' and/or 3' nucleotides (Adams, M., et al., 1991, Science, 252, 1651-1656). Each EST sequence read would generate; on average, a 500bp tag per transcript. The number of identical or overlapping ESTs would indicate the relative level of gene expression activity. Though this is an effective approach to identifying genes, it is prohibitively expensive to tag every transcript in a transcriptome. In practice, sequencing usually ceases after 10,000 or fewer ESTs are obtained from a cDNA library where millions of transcripts might be cloned.

To increase the efficiency of sequencing and counting large numbers of transcripts, Serial Analysis of Gene Expression (SAGE) (Velculescu, V. E., et al., 1995, Science, 270, 484-487) and the recent Massively Parallel Signature Sequencing (MPSS) technique (Brenner S, et al., 2000, Nature Biotechnology, 18, 630-634) were developed based on how a short signature sequence (14-20bp) of a transcript can be sufficiently specific to represent that gene.

Experimentally, one short tag per transcript can be extracted from cDNA. Such short tags can be sequenced efficiently either by a concatenation tactic (as for SAGE), or by a hybridization-based methodology for MPSS. For example; in SAGE, multiple tags are concatenated into long DNA fragments and cloned for sequencing. Each SAGE sequence readout can usually reveal 20-30 SAGE tags. A modest SAGE sequencing effort of less than 10,000 reads will have significant coverage of a transcriptome. Transcript abundance is measured by simply counting the numerical frequency of the SAGE tags.

In theory, short DNA tags of about 20bp can be specifically mapped to a single location within a complex mammalian genome and uniquely represent a transcript in the content of whole transcriptome. However, in reality, there still exist a large number of "ambiguous" SAGE tags (14-21 bp) and MPSS tags (17bp) that have multiple locations in a genome, and may be shared by many genes.

A recent sequencing technology is that of "pyrosequencing" or "454" technology (Margulies et al, 2005). In recently published findings, a "454" sequencing run can simultaneously read 300,000 templates and achieve a 100-fold efficiency increase and 10-fold cost reduction compared with current sequencing instruments. However; each "454" sequencing read can only read about 100 bp, seriously limiting its potential because it is difficult to sequence large contiguous stretches of DNA.

Hence; the main problems in the current art of genomic sequencing are of ambiguity when using short nucleotide tags to represent gene transcripts, and the limitation of current multiplex sequencing technologies to reading short stretches of nucleotides.

### Summary of the Invention

The present invention solves the problems mentioned above by providing a new method of manipulating nucleic acids. More specifically, the present invention relates to manipulation of nucleic acids. In particular, the invention relates to methods for the preparation of nucleotide fragments by concatenation.

In one aspect, the present invention provides a new method of concatenation of nucleotide fragments. In particular, there is provided a length-controlled concatenation of nucleotide fragments such that concatemers having a desired number of nucleotide fragments; or having a particular length, may be prepared. The present disclosure also provides molecules and components prepared by the method. The nucleotide fragment according to the invention may comprise at least one ditag and/or at least one tag. Accordingly, a concatenation of at least two nucleotide fragments may comprise at least two concatenated ditags and/or tags

Accordingly, there is provided a method of length-controlled concatenating nucleotide fragments, as defined in the claims.

According to one aspect, the repetition of concatenation yields a doubling of the number of concatenated nucleotide fragments.

The method may further comprise treating the at least one oligonucleotide to produce at least one oligonucleotide with two ligatable ends and allowing the oligonucleotide to self-circularize at the ligatable ends. The method may further comprise selecting the at least one circularized oligonucleotide and/or amplifying the oligonucleotide. The method may further comprise treating the circularized and/or amplified oligonucleotide to produce at least one oligonucleotide having one ligatable end and one non-ligatable end, and allowing at least two oligonucleotides to ligate at the ligatable ends to form a concatemer comprising at least two concatenated oligonucleotides.

The method may further comprise: (a) treating the at least one oligonucleotide to produce at least one oligonucleotide having two ligatable ends compatible with each other, and allowing the oligonucleotide to self-circularize at its ligatable ends; (b) selecting the at least one self-circularized oligonucleotide; (c) optionally amplifying the selected oligonucleotide; (d) treating the oligonucleotide (from step b or c) to produce at least one oligonucleotide with one ligatable end and one non-ligatable end; and (e) allowing two oligonucleotides to ligate at the ligatable ends to form a concatemer comprising at least two concatenated oligonucleotides.

In another embodiment, the method comprises the steps of (a) providing at least one oligonucleotide comprising at least one nucleotide fragment, wherein the oligonucleotide has ligatable ends; (b) allowing the at least one oligonucleotide to self-circularize at its ligatable ends; (c) selecting and optionally amplifying the at least one self-circularized oligonucleotide; (d) treating the selected circularized oligonucleotides with at least one restriction enzyme to obtain at least one oligonucleotide with one ligatable end and one non-ligatable end; and (e) concatenating at least two oligonucleotides at the ligatable ends to form a concatenated oligonucleotide comprising two nucleotide fragments.

For the embodiments in this aspect of the invention, the nucleotide fragments, oligonucleotide(s) and/or concatemer(s) may be amplified. The amplification may be by bacterial amplification, by rolling circle amplification, and/or by polymerase chain reaction. The method may comprise repeating the steps one or more times to obtain concatemers having desired lengths and/or number of oligonucleotides or nucleotide fragments. The repeating may result in a doubling of the number of oligonucleotides in the concatemers. The ligatable end of each fragment may be a palindromic cohesive end. The ligatable end and/or the non-ligatable end may be located in at least one adaptor. The adaptor may be part of a plasmid or vector. The nucleotide fragment may comprise at least one ditag, the ditag comprising at least one first tag comprising a 5' terminus and at least one second tag comprising a 3' terminus of a polynucleotide. Each nucleotide fragment of the concatemer may have an orientation opposite to the orientation of a nucleotide fragment positioned upstream and/or downstream. The method may further comprise sequencing the concatemer. The sequencing may be by any suitable method, for example, by pyrosequencing.

Also disclosed herein is an isolated concatemer (one oligonucleotide or at least two concatenated oligonucleotides) comprising at least two nucleotide fragments, wherein each fragment has at least one ligatable end and one non-ligatable end, and the fragments are ligated at the ligatable ends to form the concatemer. The ligatable ends may be palindromic cohesive ends. The fragment may comprise at least one ditag, the ditag comprising at least one first tag comprising a 5' terminus and at least one second tag comprising a 3' terminus of a polynucleotide. Each nucleotide fragment of the concatemer may have orientation opposite to the orientation of a .nucleotide fragment positioned upstream and/or downstream. The concatemer may be inserted into a plasmid or vector. The polynucleotide may be DNA or RNA.

### Brief Description of the Drawings

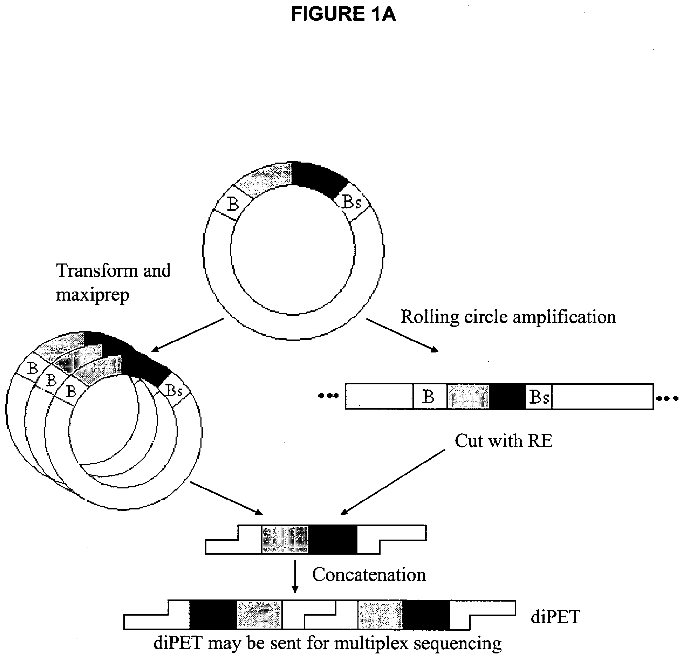

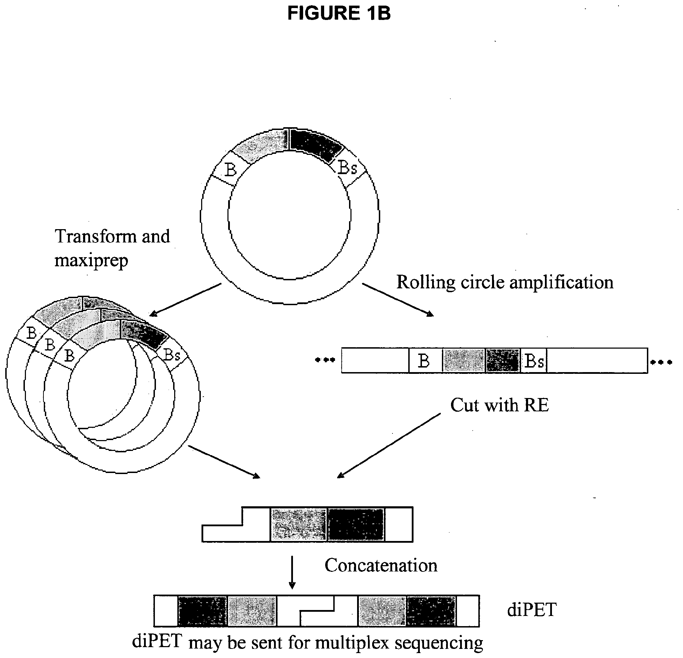

FIG.1 illustrates the overview of one embodiment of the method of the present invention for preparing concatenated nucleotide fragments (concatements). Each nucleotide fragment comprises a ditag. The ditag comprises a first tag comprising a 5' terminus (gray in the figure) and a second tag comprising a 3' terminus (black in the figure) of a polynucleotide (for example, a full-length cDNA). The ditag may have one end sticky or ligatable and the other end non-sticky (FIG 1A) or blunt-ended (may be either ligatable or non-ligatable) (FIG. 1B) The diPET as shown in FIG. 1A may be further ligated to nucleotide fragments with complementary ends and the diPET shown in FIG.1B may be further ligated to nucleotide fragments with ligatable (blunt-ended, phosphorylated) ends. The two concatenated nucleotide fragments obtained comprise two concatenated ditags. In the figure, it is shown that the first and second ditag of each two concatenated nucleotide fragments have opposite orientation. The ditags are suitable for sequencing by large scale parallel sequencing methods, such as "454" sequencing. The ditags may be prepared by using single paired-end ditag (PET) plasmids or from insertion of other DNA sequences wherein the 5' and 3' termini are of interest. Mme1 sites are present, but not shown on the plasmid.

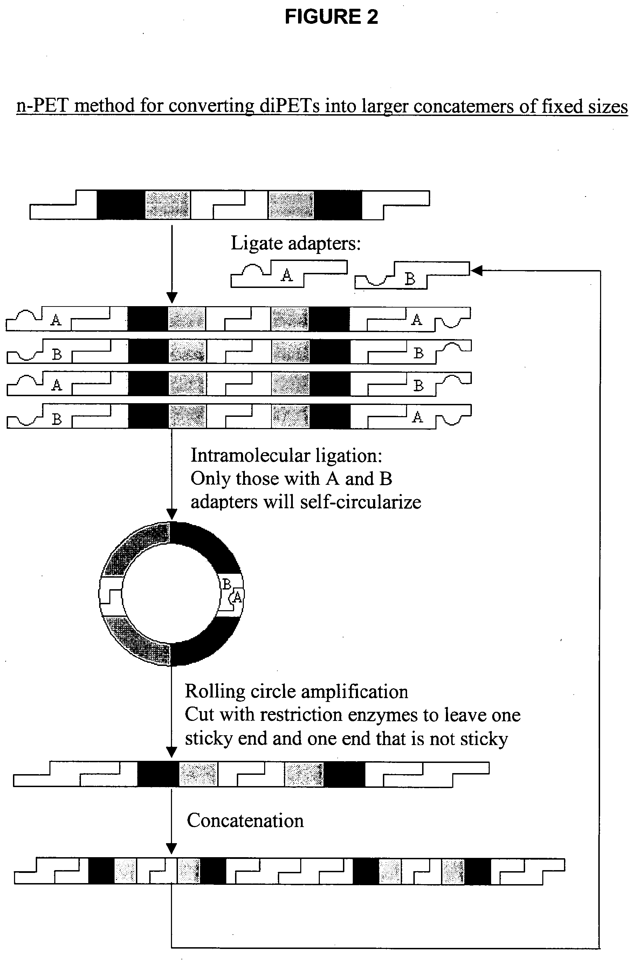

FIG.2 illustrates the overview of another embodiment of the method of the present invention for preparing concatemers comprising n-PETs (where n=4,8,16,32....), allowing the scaling up of the number of paired-end ditags (PETs). The oligonucleotides in the concatemers may be added in a precise manner as the capacity of the sequencing technology used increases. Two different types of adaptors are ligated to the ends of the diPET. These adaptors will contain the different restriction sites necessary for subsequent restriction digestion. The adaptors are ligated, such that only those diPETs with different adaptors ligated on will be circularized, and thus selected by an optional exonuclease treatment. Rolling circle amplification is performed to amplify the DNA. The DNA is then cut with the appropriate restriction enymes to generate a sticky end and an end that is not sticky, such that ligation may be used to form an n-PET. The cycle may then be repeated as desired to generate larger n-pets. Amplification and selection by PCR is also possible.

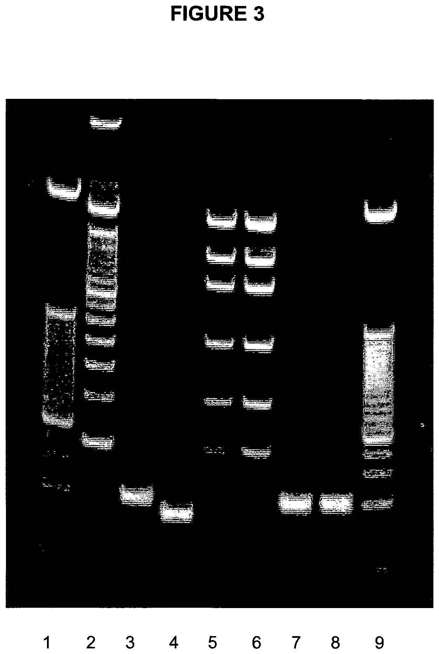

FIG.3 illustrates electroeluted PETs in a PAGE gel. Lanes 1 and 9: Invitrogen 25 bp ladder. Lane 2: Invitrogen 100 bp ladder. Lane 3: BseR1 and BamH1 cut PETs from control library. Lane 4: BseR1 and BamH1 cut PETs from experimental library. Lane 5: 2 ul Invitrogen Low Mass ladder. Lane 6: 4 ul Invitrogen Low Mass ladder. Lane 7: Blunted BseR1 and BamH1 cut PETs from control library. Lane 8: Blunted BseR1 and BamH1 cut PETs from experimental library.

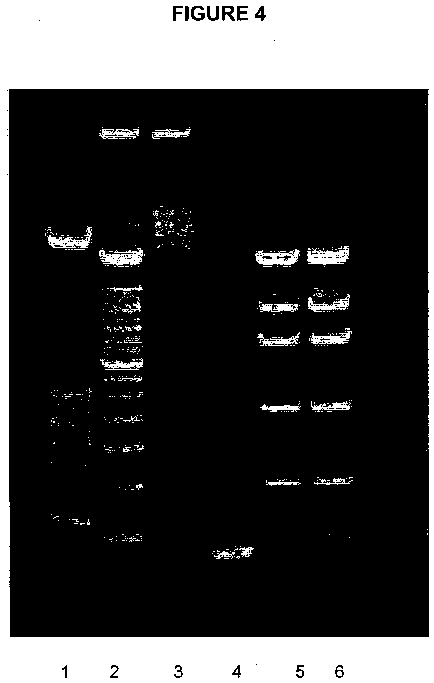

FIG. 4 illustrates electroeluted diPETs in a PAGE gel. Lane 1: Invitrogen 25 bp ladder. Lane 2: Invitrogen 100 bp ladder. Lane 3: Ligation product of control library; as expected, this library formed concatemers. Lane 4: Ligation product of experimental library - this library formed length-controlled diPETs, as can be seen by the single clear, sharp band. Lane 5: 2 ul of Invitrogen Low Mass ladder. Lane 6: 4 ul of Invitrogen Low Mass ladder.

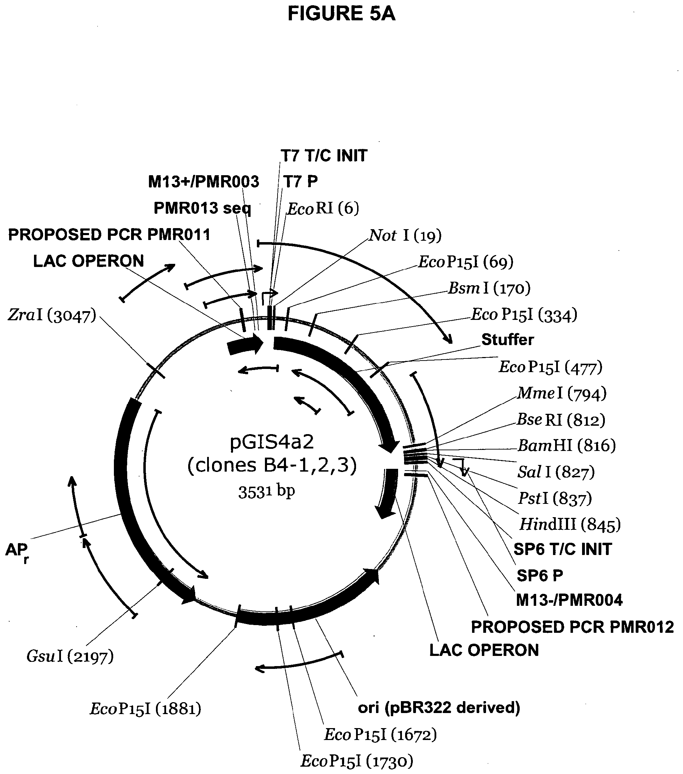

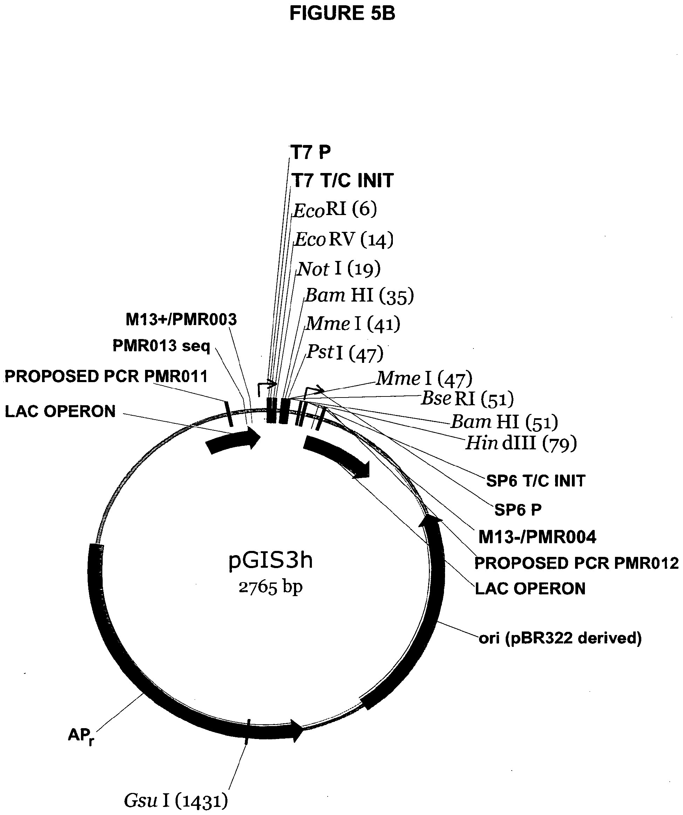

FIG. 5 illustrates two examples of vectors used in the method of the present invention. FIG. 5A is the pGIS4a2 vector and FIG. 5B is the pGIS3h vector.

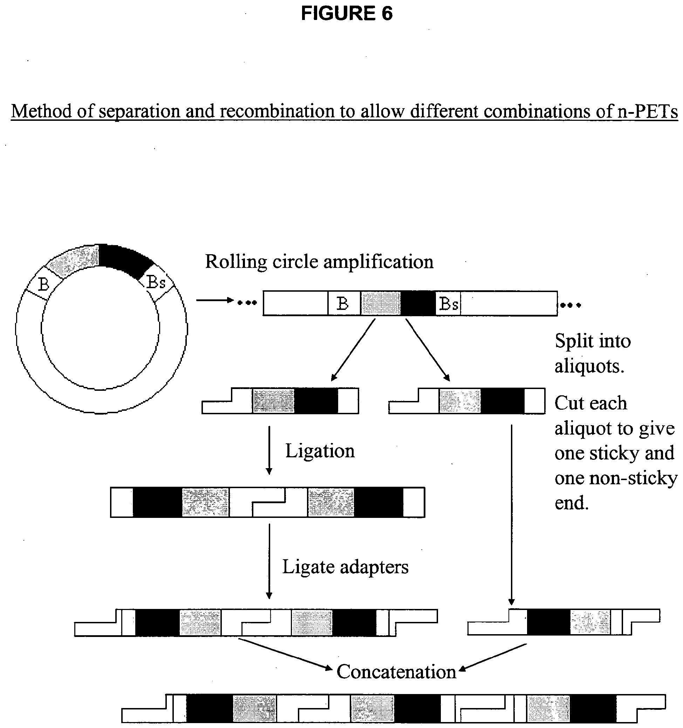

FIG. 6 illustrates concatenation of ditags to obtain a concatemer of a desired length for sequencing.

### Sequence numbering of primers, adaptors and vectors

| | |
|---|---|
| SEQ ID NO:1: Gsul-oligo dT primer: 5'-GAGCTAGTTCTGGAGTTTTTTTTTTTTTTTTVN-3' | |
| | |
| SEQ ID NO:2: GIS-(N)6 adapter upper strand: 5'-CTAAACTCGAGGCGGCCGCGGATCCGACNNNNNN-3' | |
| | |
| SEQ ID NO:3: GIS-(N)6 adapter lower strand: 5'-p-GTCGGATCCGCGGCCGCCTCGAGTTT-3' | |
| | |
| SEQ ID NO:4: GIS-(N)5 adapter upper strand: 5'-CTAAACTCGAGGCGGCCGCGGATCCGACGNNNNN-3' | |
| | |
| SEQ ID NO:5: GIS-(N)5 adapter lower strand: 5'-p-GTCGGATCCGCGGCCGCCTCGAGTTT-3' | |
| | |
| SEQ ID NO:6: palindromic upper strand: 5'-GTCGGATCCGAC-3' | |
| | |
| SEQ ID NO:7: palindromic lower strand 5'- GTCGGATCCGAC-3' | |
| | |
| SEQ ID NO:8: n-PET TT-tailed adaptor (PMR 011) - upper strand: 5' GCTTGTAAGCTACTCCTCGATGTGCTGCAAGGCGATTAAG 3' (40 nt) | |
| SEQ ID NO:9: n-PET TT-tailed adaptor (PMR 011) - lower strand: 3' TTCGAACATTCGATGAGGAGCTACACGACGTTCCGCTAATTC 5' (42 nt) | |
| | |
| SEQ ID NO:10: n-PET TT-tailed adaptor (PMR 012)- upper strand: 5' GCTTGTAAGCTACTCCTCAGCGGATAACAATTTCACACAGG 3' (41 nt) | |
| | |
| SEQ ID NO:11: n-PET TT-tailed adaptor (PMR 012) - lower strand: 3' TTCGAACATTCGATGAGGAGTCGCCTATTGTTAAAGTGTGTCC 5' (43 nt) | |
| | |
| SEQ ID NO:12: PMR011: | |
| 5' GATGTGCTGCAAGGCGATTAAG 3' | (22 nt) |
| 5' ends are phosphorylated | |
| | |
| SEQ ID NO:13: PMR012 | |
| 5' AGCGGATAACAATTTCACACAGG 3' | (23 nt) |
| 5' ends are phosphorylated | |
| | |
| SEQ ID NO:14: pGIS4a2 sense(FIG. 5A) | (3531 bp) |
| See sequence listing. | |
| | |
| SEQ ID NO:15: pGIS4a2 antisense(FIG. 5A) | (3531 bp) |
| See sequence listing. | |
| | |
| SEQ ID NO:16: pGIS3h sense (FIG. 5B) | (2765 bp) |
| See sequence listing. | |
| | |
| SEQ ID NO:17: pGIS3h antisense (FIG. 5B) | (2765 bp) |
| See sequence listing. | |
| | |
| SEQ ID NO:18; diPET from pGIS4a diPETtinq sense | (82bp) |
| | |
| SEQ ID NO:19; diPET from pGIS4a diPETtinq antisense | (82bp) |
| | |
| SEQ ID NO:20; diPET from pGIS3h diPETtinq sense | (90bp) |
| | |
| SEQ ID NO:21; diPET from pGIS3h diPETtinq antisense | (90bp) |

### Detailed Description of the Invention

### Definitions.

Restriction enzyme - A restriction enzyme (or restriction endonuclease) is an enzyme that cuts double-stranded DNA. The enzyme makes two incisions, one through each of the phosphate backbones of the double helix without damaging the bases. The chemical bonds cleaved by the enzymes may be reformed by other enzymes known as ligases, enabling restriction fragments obtained from different chromosomes or genes to be joined or spliced together, provided their ends are complementary or compatible. Type II enzymes recognize specific nucleic sequences (recognition sites) and cut DNA at defined positions close to or within their recognition sequence sites. They produce discrete restriction fragments and distinct gel banding patterns. Type II enzymes cleave outside of their recognition sequence to one side. MmeI, as well as most of the type IIs restriction enzymes, produce variable end lengths. Dunn et al (2002) showed that MmeI can cut 18/20 or 19/21 bases away in a rough proportion of 1:1. Type III enzymes are also large combination restriction-and-modification enzymes. They cleave outside of their recognition sequences and require two such sequences in opposite orientations within the same DNA molecule to accomplish cleavage. Homing endonucleases are rare double-stranded DNases that have large, asymmetric recognition sites (12-40 base pairs) and coding sequences that are usually embedded in either introns (DNA) or inteins (proteins). Restriction enzymes may make cuts that leave either non-sticky (blunt) end or sticky (ligatable) ends with overhangs. A sticky-end fragment can be ligated not only to the fragment from which it was originally cleaved, but also to any other fragment with a complementary, compatible, cohesive or sticky end. As such, ends produced by different enzymes may also be compatible. Many type II restriction enzymes cut palindromic DNA sequences. If a restriction enzyme cuts a nondegenerate palindromic cleavage site, all the ends produced are compatible. A "palindromic" sequence is found where the sequence on one strand reads the same in the opposite direction on the complementary strand, allowing nucleic sequences cleaved to obtain palindromic cohesive ends can self-circularize when the two ends on the same strand mate. The meaning of "palindromic" in this context is different from its linguistic usage. For example, the sequence GTAATG is not a palindromic DNA sequence, while the sequence GTATAC is. Examples of restriction enzymes leaving cohesive or sticky ends include BamH1, EcoR1 and HindIII. An example of restriction enzymes leaving blunt, non-cohesive or non-sticky ends is Alul. Under the present invention, an end of a nucleic acid strand is said to be ligatable or capable of being ligated if it has a complementary, compatible, cohesive or sticky end or phosphorylated blunt end. An end of a nucleic is said not to be ligatable or not capable of being ligated if it and the other strand of nucleic acid both have dephosphorylated ends, or if it does not have an end that another strand of nucleic acid is complementary, compatible, cohesive or sticky to. Also, a restriction enzyme name (such as EcoR1) can also refer to the nucleic acid sequence or recognition site recognized by the enzyme as readily understood in the context in which the enzyme name or recognition site appears.

Nucleotide - a phosphoric ester of nucleoside; the basic structural unit of nucleic acids (DNA or RNA). Nucleotides form base pairs - one of the pairs of chemical bases joined by hydrogen bonds that connect the complementary strands of a DNA molecule or of an RNA molecule that has two strands; the base pairs are adenine with thymine and guanine with cytosine in DNA and adenine with uracil and guanine with cytosine in RNA. Nucleotides may be joined with or concatenated with other nucleotides. The term nucleotide may be used interchangeably with the term nucleic acid. Each nucleotide possesses a 5' end and a 3' end and accordingly, a strand of nucleic acids may also possess a 5' end and a 3' end. The end regions of a strand of nucleic acids may be referred to as the 5' terminus and the 3' terminus respectively. Nucleic acid sequences are conventionally read in the 5' to 3' direction which gives the orientation of the nucleotides. Short strands of nucleotides are referred to as oligonucleotides while longer strands are referred to as polynucleotides. Under the present invention, an oligonucleotide can comprise at least one nucleotide fragment, tag or ditag. A fragment is a length of nucleic acids obtained, derived or prepared from a longer length of nucleic acids. As such, a fragment can comprise at least one tag or ditag and can represent a larger nucleic acid molecule. Under the present invention, a polynucleotide can refer to a gene, a message RNA transcript of a gene, parts of a gene or a cDNA sequence representing a gene. With reference to a first oligonucleotide, a second oligonucleotide may be referred to as being "upstream" from it; if the second oligonucleotide is positioned nearer to the 5' end of the first oligonucleotide or "downstream" if the second nucleotide is nearer to the 3' end of the first oligonucleotide.

Concatemer - It is composed by at least two nucleotide monomers sequences linked end to end, optionally separated by a linker or spacer. For the purpose of the present invention, a concatemer comprises at least two tags, two ditags, two nucleotide fragments or two oligonucleotides prepared according to the method of the invention. In the present invention, two oligonucleotides may be concatenated such that the 5' to 3' orientation of one nucleotide fragment in an oligonucleotide is opposite to the orientation of an adjacent nucleotide fragment positioned upstream or downstream of it.

Plasmid - With the term vector or recombinant vector it is intended a plasmid, virus or other vehicle known in the art that has been manipulated by insertion or incorporation of the ditag genetic sequences. Such vectors contain a promoter sequence that facilitates the efficient transcription of the inserted sequence. The vector typically contains an origin of replication, a promoter, as well as specific genes which allow phenotypic selection of the transformed cells. Vectors suitable for use in the present invention include for example, pBlueScript (Stratagene, La Jolla, CA); pBC, pZErO-1 (Invitrogen, Carlsbad, CA) and pGEM3z (Promega, Madison, WI) or modified vectors thereof, as well as other similar vectors known to those of skill in the art. The pGEM vectors have also been disclosed in US 4,766,072. In the present invention, the plasmid PGIS4a2 (clone B4-1) (FIG. 5A) was used.

Obtain, derive, prepare - to use molecular biology and genetic engineering and manipulation techniques on biological material such as nucleic acids and proteins to confer upon the material certain desired characteristics. The terms obtain, derive and prepare may be used interchangeably under the present invention.

Amplification - increasing the copy number of nucleic acids. One method commonly used is that of polymerase chain reaction (PCR). Other amplification methods known to a skilled person such as bacterial amplification or rolling circle amplification may also be used.

Tag - A tag or signature is an identifiable sequence of nucleic acids. It may refer to either the 5'- or 3'-most terminal nucleic acid sequence (terminus; of any length but usually 18-20 bp) derived from any contiguous DNA region. The terms tag and signature may be used interchangeably under the present invention. Under the present invention, a single tag signature (about 20bp) from each of two nucleotide fragments may be ligated to form a "tag1-linker-tag2" (also referred to as "first tag-linker-second tag) paired end ditag (PET) structure. Another possible arrangement is a linker-tag-tag-linker structure where a linker flanks a tag (that is,a linker is positioned upstream and/or downstream to at least one of the tag).

Linker - A linker is an artificial sequence of nucleic, usually containing one or more restriction enzyme recognition sites.

Ditag - A short (usually 12-60 bp) strand of nucleotides comprising at least one tag or signature derived from a longer strand of nucleotides. A ditag may be prepared according to US 20050255501 and/or US 20050059022. A ditag may comprise either or both the 5' end region (also indicated as 5' tag) and 3' end region (also indicated as 3' tag) of a nucleic acid molecule. Under the present invention, a single tag signature (about 20bp) from each of two nucleotide fragments may be ligated to form a "tag1-linker-tag2" (also referred to as "first tag-linker-second tag) paired end ditag (PET) structure. When two paired end ditags (PET) each comprises a 5' tag and a 3' tag of each nucleotide fragment, for example the structure of "PET1-linker-PET2", is called a diPET.

Sequencing - The methods used to determine the order of constituents in a biopolymer, in this case, a nucleic acid. Sequencing techniques used include Sanger method and modified variations thereof, as well as pyrosequencing or the "454 method" of sequencing.

In the following description, details are provided to describe the embodiments of the present invention. It shall be apparent to one skilled in the art, however the invention may be practiced without such details. Some of the details may not be described at length so as not to obscure the invention.

For the performance of the methods of the present invention for a particular embodiment, any description disclosed for the purpose of carrying out other embodiments of this invention may also be used. In particular, technique(s), reagents, experimental conditions, restriction sites, enzymes, vectors, primers, and the like. In particular, it will be evident to any skilled person how to adapt techniques and material disclosed for the other embodiments to the present embodiment of the invention.

Bibliographic references mentioned in the present specification are for convenience listed in the form of a list of references and added at the end of the examples.

Standard molecular biology techniques known in the art and not specifically described were generally followed as described in standard molecular biology reference books such as Molecular Cloning: A Laboratory Manual by Sambrook and Russell, Third Edition, 2001, published by Cold Spring Harbor Laboratory Press.

### Description

The present invention relates to a new method of manipulating nucleic acids. More specifically, the present invention relates to manipulation of nucleic acids by concatenating them. In particular, the invention relates to methods for the preparation of ditags and/or tags representing polynucleotides by concatenation.

In one aspect, the present invention provides a method for length-controlled concatenation of signature tags representing polynucleotides such that concatemers having desired number of ditags and/or tags or having a particular length may be prepared. The disclosure also provides molecules and components prepared by the method.

Accordingly, there is provided a method of length-controlled concatenating nucleotide fragments, as defined in the claims (FIG. 1). This method may be repeated one or more times.

In one aspect, the repetition of concatenating results in a doubling of the number of concatenated nucleotide fragments.

There is also disclosed a method (see FIG.2) comprising the steps of (a) providing at least one oligonucleotide comprising at least one nucleotide fragments, preferably comprising at least two nucleotide fragments, wherein the oligonucleotide has ligatable ends; (b) allowing the at least one oligonucleotide to self-circularize at its ligatable ends; (c) selecting the at least one self-circularized oligonucleotide; (d) treating the selected circularized oligonucleotide with at least one restriction enzyme to obtain at least one oligonucleotide with one ligatable end and one non- ligatable end; and (e) concatenating at least two oligonucleotides at the ligatable ends to form a concatemer of at least two oligonucleotides or at least two nucleotide fragments. In particular, the provided oligonucleotide in step (a) may comprise at least two concatenated nucleotide fragments, and the obtained concatenated oligonucleotide in step (e) comprises at least two four-concatenated nucleotide fragments (as shown in FIG.2). Each nucleotide fragment may comprise at least one ditag, the ditag comprising at least one first tag comprising a 5' terminus and at least one second tag comprising a 3' terminus of a polynucleotide. In one embodiment, the first ditag of nucleotide fragment has opposite orientation to the second ditag of the same nucleotide fragment. Accordingly, each nucleotide fragment of the oligonucleotide has the opposite orientation to a nucleotide fragment positioned upstream and/or downstream. This method may be repeated one or more times. In one aspect, each repeating of concatenating results in a doubling of the number of concatenated nucleotide fragments.

### Multiplex Sequencing

The "454" sequencing or pyrosequencing technology has been developed for genomic DNA sequencing but each read can only read 100 bp. This restriction places a limit on the number of tags that can be used. When increasing the number of tags that can be accommodated, the length of each tag will necessarily have to be shortened; concomitantly decreasing specificity and increasing ambiguity. In contrast; if ditags are used in the method of the present invention to prepare length-controlled concatemers, each read will reveal hundreds or even thousands of base pairs of information as demarcated by the ditags. Furthermore, using diPETs as templates, the sequencing throughput and capacity of "454" sequencing are increased two-fold. Hence, the method of the present invention, when applied to preparing concatemers for sequencing and coupled with multiplex sequencing methods, is at least 500-fold more efficient than any of the currently existing methods for DNA sequencing analysis. This method of length-controlled concatenation can be extended for a number of cycles to prepare concatemers having multiple ditags and may be applied to generate desired lengths of concatemers of any kind of tag fragment such as SAGE or "454" multiplex sequencing technology. In addition, the method of the present invention may be applied to cDNA sequencing and ChIP DNA fragment sequencing as well as other sequencing technologies such as SAGE or MPSS.

To overcome the problems and limitations of the current art, the present invention provides, in one embodiment, a method for length-controlled concatenation of signature tags representing polynucleotides such that concatemers having desired number of tags or ditags and having a particular length may be prepared. Such concatemers may be used in various sequencing technologies.

### Tags and ditags

The ditags of the present invention may be prepared or obtained from the paired end ditagging (PET) strategy (US 20050255501). For the purpose of the present application, a tag is a fragment obtained from a nucleic acid molecule and represents the polynucleotide from which the tag was obtained or derived from. The polynucleotide which is intended to shrink or represent may be RNA, mRNA, genomic DNA, full-length cDNA, or cDNA.

Under the present invention, two tags or fragments that are present in an oligonucleotide of the present invention may also be called a ditag. Like fragments or tags, a ditag is shorter than the original nucleic acid molecule from which it originates or which it represents. Preferably, the ditag must be much shorter than the original nucleic acid molecule. As consequence of the "shrinking", the ditag may essentially comprise either or both the 5' end region (also indicated as 5' tag) and 3' end region (also indicated as 3' tag) of the original nucleic acid molecule. Hence, the portion of the original nucleic acid molecule that is between or inside the 5' tag and 3' tag is not included in the ditag. The ditag according to the invention retains the most informative features of the original nucleic acid molecule, namely: the start and the end signatures of the nucleic acid.

The 5' tag and 3' tag forming the ditag may have the same or different size. Preferably, they have the same number of nucleotides. The ditag may be of any size, but needs to be meaningful and advantageous over the size of the parental sequence from which it is derived. The preferred size of a tag or ditag is determined by genome complexity. For a bacterial genome a tag from about 8 bp to about 16 bp may be sufficient whereas for a complex genome like the human genome, a 16-20 bp tag (which results in a 32-40bp ditag) may be considered. In general, the size of the ditag is from about 12-60 bp.

For the purpose of the present application, the terms 5'-terminus, 5'-end and 5'-tag are equivalent to each other and may be used interchangeably. In the same way, the terms 3'-terminus, 3'-end and 3'-tag are equivalent to each other and may be used interchangeably. In an original nucleic acid molecule or polynucleotide, or portion inside a nucleic acid molecule or polynucleotide that one intends to reduce or represent, each 5'-end and 3'-end represents a region or portion closest to the extremity; farthest from the middle region of the nucleic acid molecule or polynucleotide. With a 5' or 3' terminus of a polynucleotide, it is understood that any region, fragment or whole piece of a polynucleotide that comprises the actual 5' or 3' terminus of the polynucleotide are included.

Each ditag comprises sufficient information to characterize a specific polynucleotide. Hence, the ditag is representative of the structure and identity of the polynucleotide.

### Concatenating oligonucleotides, nucleotide fragments, ditags or tags

While fragments or tags of sufficient length may be prepared, obtaining concatemers of a predetermined length involve technical difficulties. Current methods for tag concatenation randomly generate concatemers with a range of lengths: monomers, dimers, trimers and so forth. In the current art, such concatemers have to be run in an electrophoresis gel to separate the concatemers, and the desired size of concatemers are excised from the gel - a laborious task. This technique is inefficient and requires large amounts of input DNA.

The present invention provides a method of length-controlled concatenation to generate oligonucleotides of a predetermined length. The present invention achieves this by preparing fragments; ditags or tags with a ligatable end and a non-ligatable end. Using this technique, a compatible, cohesive or sticky end on one fragment or tag will join or ligate to another sticky end on another fragment or tag. When this happens, the non-sticky ends will not permit further ligation and concatenation stops. This technique is further illustrated in the examples below. Should ligatable ends not be found readily in the fragments or tags, suitable adaptors possessing the appropriate restriction enzyme recognition sites may be ligated to the fragments or tags. The ligatable ends may be palindromic ends.

Alternatively, if two different enzymes cannot be used in order to yield one end that is palindromic and another end that will not self-ligate, the enzymes may be used sequentially, and after the first restriction digest, one end may be "blocked" by dephosphorylation or other means, such as attachment to a solid substrate.

### The diPET embodiment

In this embodiment, tags with the single PET structures in the plasmids are flanked by the restriction enzyme recognition sites for a cohesive palindromic enzyme and an enzyme leaving a blunt end. For example, the two sites may be BamH1 (B) at one side and BseR1 (Bs) at the other side (FIG. 1A), such that the BamH1 cut leaves a palindromic cohesive end compatible to each other, while the BseR1 cut is designed to leave an AA residual or any non-palindrome sequence, which does not match to itself. The PETs may be amplified, whether by bacterial amplification, rolling circle amplification, or other amplification methods (FIGS. 1A and 1B). The PETs are then first cut with one restriction enzyme, in this embodiment BseR1, followed by cutting with a different restriction enzyme, in this embodiment, BamH1.

Released PETs may be purified by any suitable method such as gel purification. Upon exposure to another similarly-prepared PET, any two of the BamH1 cohesive ends will find each other and mate, resulting in oligonucleotide concatemers having a dimer PET or diPET structures with two non-palindromic ends on each side of the oligonucleotide (FIG. 1 B). These non-palindromic ends prevent further ligation with other PETs, stopping concatenation.

This embodiment gives rise to a diPET oligonucleotide concatemer made of two PETs of about 80bp: which is below the maximum capacity of the current "454" sequencing system.

While the preferred embodiment uses PETs, any tag can also be turned into "diPETs" by this method. It is also preferable to use at least one type IIs restriction enzyme, such as BseR1, as this will minimize the length of the border sequences. As long as the cut sites of the type IIs restriction enzyme are different, just one type IIs restriction enzyme site may be used.

### The n-PET embodiment

This method allows the creation of DNA sequences consisting of n numbers of PETs (where n = 4, 8, 16, 32....), allowing scaling up of the number of
PETs added in a length-controlled manner to suit the capacity of the particular sequencing technology used.

Referring to FIG. 2, two different types of adaptors (labeled as A and B) are ligated to the ends of the diPET. These adaptors contain the different restriction sites necessary for restriction digest later. The adaptors are ligated, such that only those diPETs with different adaptors ligated will be circularized by self-circularization, and thus selected by an exonuclease treatment. Rolling circle amplification is performed to amplify the DNA. Alternatively, amplification and selection by PCR is also possible as the adaptor sequences are known. The DNA is then cut with the appropriate restriction enzymes to generate a palindromic end and a non-palindromic end, such that ligation may be used to form a 4-PET oligonucleotide. The cycle may then be repeated as desired to generate larger oligonucleotide concatemers comprising n-PETs.

Alternatively, two different types of adaptors may be ligated to the ends of the diPET, following which PCR is performed. Adaptors which are compatible will snap together, preventing PCR from taking place, allowing only adaptors which are different to be amplified. The DNA may then be cut with the appropriate restriction enzymes, and the cycle repeated if desired.

It may be seen, in the diPET and n-PET embodiments, that concatenation in these embodiments results in the 5' to 3' orientation of a nucleotide fragment, tag or ditag being opposite to another nucleotide fragment, tag or ditag adjacent (that is, positioned upstream or downstream) to it (FIGS. 1 and 2). Also seen in this embodiment is the doubling of the number of diPETs concatenated with each repeat of the ligation step.

While the embodiments of the method of the present invention above make use of fragments or tags that have ligatable ends, the method of the present invention can also make use of fragments that do not have ligatable ends by adding suitable adaptors to them. Alternatively, If two different enzymes cannot be used which result in one end that is palindromic and another end that will not match to itself, the enzymes may be used sequentially, and after the first restriction digest, one end may be "blocked" by dephosphorylation or other means, such as attachment to a solid substrate.

The embodiments of the method for fragments, ditags or tags may also be applied to oligonucleotides or oligonucleotide concatemers. For example, suitable adaptors may also be ligated to oligonucleotide concatemers to allow them to ligate to another nucleotide fragment, tag or oligonucleotide with a compatible cohesive end.

Accordingly, in another embodiment, the method may further comprise the steps of treating the at least one oligonucleotide to produce at least one oligonucleotide having one ligatable end and one non-ligatable end, and allowing the oligonucleotide to ligate with a further oligonucleotide or nucleotide fragment to form a concatemer comprising at least two oligonucleotides or at least one oligonucleotide and at least one nucleotide fragment.

In another embodiment, the method may further comprise: (a) treating the at least one oligonucleotide to produce at least one oligonucleotide having two ligatable ends compatible with each other, and allowing the oligonucleotide to self-circularize; (b) selecting the at least one self-circularized oligonucleotide; (c) optionally amplifying the selected oligonucleotide; (d) treating the oligonucleotide from the previous step to produce at least one oligonucleotide with one ligatable end and one non- ligatable end; and (e) allowing two oligonucleotides to ligate at the ligatable ends to form a concatemer comprising at least two oligonucleotides.

There is also provided an isolated oligonucleotide comprising at least two nucleotide fragments, wherein each fragment has at least one ligatable end and and one non-ligatable end, and the fragments are ligated at the ligatable ends to form the oligonucleotide. Preferably, the ligatable ends are palindromic cohesive ends. The concatemer or concatenated oligonucleotide(s) according to the invention comprises at least one nucleotide fragment, the fragment comprising at least one ditag, the ditag comprising at least one first tag comprising a 5' terminus and at least one second tag comprising a 3' terminus of a polynucleotide. The polynucleotide may be a full-length cDNA or one or more exons. Accordingly, the ditag may be representative of the full-length cDNA. In one embodiment; the concatemer or oligonucleotide according to the invention has each nucleotide fragment (or ditag) in an orientation opposite to the orientation of a nucleotide fragment (or ditag) positioned upstream and/or downstream. The concatemer, oligonucleotide, nucleotide fragment, ditag or tag according to the invention may be inserted into a plasmid or vector. Accordingly, there is also provided a plasmid or vector comprising at least one concatemer, oligonucleotide, nucleotide fragment, ditag or tag according to the invention. The plasmid or vector may be inserted in a host cell.

There is also provided a kit for concatenating oligonucleotides, nucleotide fragments, ditags and/or tag according to any embodiment of the invention, comprising at least one of a restriction enzyme, at least one nucleotide fragment, ditag or tag, optionally a vector, and any reagents as herein disclosed (for instance as described in the examples) for the reaction of concatenation. The kit may further comprise illustration and/or information pertaining to the use of the kit.

There is also provided a library comprising at least a concatemer, concatenated oligonucleotides, concatenated nucleotide fragments, concatenated ditags and/or concatenated tags according to any embodiment of the invention.

### Variations

For the embodiments in this aspect of the invention, many variations in the method are possible. For example; the tags or nucleotide fragments, and/or oligonucleotides or concatemers may be amplified. The method amplification may be by bacterial amplification, by rolling circle amplification, and/or by polymerase chain reaction. The method may comprise repeating the steps one or more times to obtain concatemers of desired lengths or number of oligonucleotides. The repeating may result in a doubling of the number of oligonucleotides in the concatemers. The ligatable end of each fragment, tag or ditag may be a palindromic cohesive end. The ligatable end and/or the non-ligatable end may be located in at least one adaptor. The adaptor may be part of a plasmid or vector. The nucleotide fragment may comprise at least one ditag, the ditag comprising at least one first tag comprising a 5' terminus and at least one second tag comprising a 3' terminus of a polynucleotide. Each nucleotide fragment or tag of the concatemer may have an orientation opposite to the orientation of a nucleotide fragment positioned upstream and/or downstream. The method may further comprise sequencing the concatemer. The sequencing may be by any suitable method, for example, by pyrosequencing.

Having now generally described the invention, the same will be more readily understood through reference to the following examples that are provided by way of illustration and are not intended to be limiting of the present invention.

### Examples

### Example 1 - BseRI Linearization of Single PET plasmid

Start from the Maxiprep amplication of the vector performed after transforming single PET plasmid DNA (described in US 20050255501 and US 20050059022). Any suitable vector may be used to clone and amplify the sequence of interest, for example, pGIS4a2 (FIG. 5A; SEQ ID NO:14) or pGIS3h (FIG. 5B; SEQ ID NO:15) Usually, the yield is about 1.2 mg from 10 Q-trays, with a concentration of about 400 ng/ul

| | |
|---|---|
| 1000ug single PET plasmid DNA | 2500 ul |
| 10x NEBuffer 2 (New England Biolabs) | 400u1 |
| 100x BSA (New England Biolabs) | 40u1 |
| 4U/ul BseRI (New England Biolabs) | 1000u1 (4 fold excess) |
| Nuclease-free water | to 4000u1 |

For more efficient enzyme digestion, it is advisable to aliquot the reaction mix in tubes of 100u1 each.

BseR1 digest of single PET plasmid maxiprep - Incubate digestion at 37°C for 3 hours maximum. Perform phenol-chloroform extraction at pH 7.9 using Eppendorf 50m1 phase-lock gel.

Take note of the minimum and maximum volumes required for the various sizes of Phase-Lock gels available. To adjust the volume to the minimum volume required, add up to 1000 p1 of nuclease-free water.
50m1 phase-lock gel:
   Minimum volume: 5m1
   Maximum volume: 20m1

Isopropanol precipitation was performed next due to the large volume involved:

| | |
|---|---|
| BseRI digested single PET plasmid | 500u1 |
| 3M NaOAC pH5.2 (Ambion) | 50u1 |
| Glycoblue (Ambion) | 5u1 |
| Isopropanol (Sigma) | 500u1 |
| Total | 1055u1 |

Incubate at -20°C for an hour. Centrifuge at 13,000 RPM for 30mins at 4°C (using a Eppendorf 5415R centrifuge; the same centrifuge may be used through the rest of the protocol) and wash once with 75% ethanol (Prepared from 100% ethanol, from Merck). Resuspend the pellet DNA in a final volume of 1500u1 Qiagen Elution Buffer (EB).

For quality checking, run 400ng of BseRI cut and uncut single PET plasmid on 1% agarose gel, 110V for 40mins using the medium gel. The presence of the supercoiled single-PET plasmid would lead to the generation of PETs that are of Barn HI cohesive ends on both the 5' and 3' ends of the PETs. These would then concatenate and the final product would be a mixture of BamHI PETs concatemers and diPETs of interest. Repeat the BseRI digestion if majority of the supercoiled single PET plasmid remains uncut (FIG. 3).

If it is shown on the gel picture that there digestion is incomplete, i.e. supercoiled single PET plasmid bands are still present, it is advisable to set up another BseRI digestion.

Dephosphorylation of single PET plasmid after BseRI digestion
Note: This step is only crucial for diPET generation via the pG1 S3h vector. There is no harm in doing this for the pGIS4a vector but it is unnecessary. Save about 400ng of this sample i.e phosphorylated BseRI linearized single-PET plasmid for subsequent electrophoresis to check the quality of dephosphorylation.

| | |
|---|---|
| BseRI cut single PET plasmid | 1500u1 (-1000ug) |
| 10x Antarctic Phosphatase buffer (New England Biolabs) | 600u1 |
| 5U/ul Antarctic Phosphatase (New England Biolabs) | 1000u1 (5 fold excess) |
| Nuclease-free water | 2900u1 |
| Total | 6000u1 |

Perform isopropanol precipitation:

| | |
|---|---|
| Dephosphorylated BseRI linearized single-PET plasmid | 500u1 |
| 3M NaOAc pH 5.2 (Ambion) | 50u1 |
| Glycoblue (Ambion) | 5u1 |
| Isopropanol (Sigma) | 500u1 |
| Total | 1055u1 |

Incubate at -20°C for an hour. Centrifuge at 13,000 RPM for 30mins at 4°C. Wash Ix with 75% ethanol. Resuspend the pellet in a final volume of 500u1 EB (Qiagen).

Take about 400ng and run 1% agarose gel together with the previously saved, phosphorylated BseRI linearized single PET plasmid.

BamHI digestion to release single PETs

| | |
|---|---|
| Phosphorylated/Dephosphorylated BseRI cut single PET plasmid | 500u1(-1000ug) |
| 10x Unique BamHI buffer (New England Biolabs) | 100u1 |
| 100x BSA (New England Biolabs) | 10u1 |
| 20U/u1BamH1 (New England Biolabs) | 100u1 |
| Nuclease free water | 290u1 |
| Total | 1000u1 |

Aliquot 100u1 per reaction tube. Incubate at 37°C overnight. Perform ethanol precipitation using glycoblue to reduce the volume to allow for the ease of gel loading.
Prepare 5 tubes of the following:

| | |
|---|---|
| BamH1 digested plasmid | 200 ul |
| 100% ethanol (Merck) | 600 ul |
| 3M NaOAc pH 5.2 (Ambion) | 20 ul |
| 1M MgCl₂ (0.0225x of the sample volume) (Am bion) | 4.5 ul |
| Glycoblue (Ambion) | 2 ul. |

Incubate at -80°C for at least 30 minutes, followed by a 30 min centrifugation at 4°C. Wash with 75% ethanol once. Resuspend the pellet in a final volume of 350u1 EB. For gel loading, add 100u1 of loading dye and load 45u1/well and not more than 100ug of DNA/well on a 2% agarose gel. Electrophorese at 80V for 1.5 hours.

### Electroelution

Add 800u1 of sterile milli-Q water to Fermentas Eluta Tubes to hydrate the membrane. These will be used subsequently for electroelution.

Excise the -40bp PETs from the gel, visualizing at 365nm UV. Discard the 800u1 sterile milli-Q water and place the cut gel slice into the Eluta Tube. Use only one gel slice per Eluta Tube. Fill the tube with 1xTAE buffer without Ethidium Bromide and electroelute at 90V for 30mins and subsequent reversed polarity for 1 min in the cold room. Ensure that the Eluta Tube is free of air bubbles after adding the buffer.

Collect the eluted PETs in multiple 1.5m1 Eppendorf tubes and centrifuge at 13,000 RPM for 10mins at 4°C. This is to pellet pieces of agarose that might be present. Pipette whatever is possible and carry out isopropanol precipitation:

| | |
|---|---|
| Eluted DNA | 500u1 |
| 3M NaOAc (Ambion) | 50u1 |
| 1M MgCl2 (0.0225x of the sample volume, Ambion) | 11.25u1 |
| Glycoblue (Ambion) | 5u1 |
| Isopropanol (Sigma) | 500u1 |
| Total | 1066.25u1 |

Note: The 1 M MgCl₂ is to aid in the precipitation of small fragments of DNA. Incubate at -20°C for an hour. Centrifuge at 13,000 RPM for 30mins at 4°C. Wash Ix with 75% ethanol. Resuspend the PETs in a final volume of 12u1 EB.

### 4. Quantification of PET DNA

PET DNA can be quantified on a mini 4-20% PAGE gel (Invitrogen). Run 0.2u1 of PET DNA together with 25bp ladder and Low Mass DNA ladder (both from Invitrogen). The latter will help in the estimation of the PET DNA amount. Currently, 2u1 and 4u1 of Low Mass ladder are used in the quantification. Below shows the preparation for the ladders for per loading:

25bp ladder preparation
1 ul of 1 ug/ul 25bp ladder + 9u1 of EB + 2ul loading
dye. Total 12u1

### Low Mass ladder preparation

2u1 Low Mass Ladder Preparation
2ul of Low Mass ladder + 8u1 of EB + 2u1 loading
dye. Total 12u1

4u1 Low Mass Ladder Preparation
4u1 of Low Mass ladder + 6u1 of EB + 2ul loading
dye. Total 12u1

Run the PET DNA to be quantified together with these ladders on a 4-20% mini PAGE gel for 30mins at 200V and TBE buffer (Ambion).

Stain the PAGE gel with Sybr Green I (Molecular Probes) in TBE buffer (final concentration should be 1X, Ambion) for 10-15mins.

Note: If there is no difference in the intensity of the DNA bands for quantification, it is advisable to load less of the DNA. A rough estimation would be sufficient as the final DiPET would be run on Agilent Bioanalyzer 500/1000 kit for quantification and sizing for '454' pyrosequencing.

### DiPETting Reaction

| | |
|---|---|
| PET DNA (at least 5 ug) | 7 ul |
| 10x Sperm idine buffer (prepared in-house; below) | 1 ul |
| 5U/u1 T4 DNA ligase (Invitrogen) | 1u1 |
| Nuclease-free water to | 10u1 |

10x ligation buffer with Spermidine is made up of:
60mM Tris-HCl pH7.5 (Ambion)
60mM MgCl2 (Ambion)
50mM NaCl (Ambion)
1 mg/m1 BSA (New England Biolabs)
70mM Beta-mercaptoethanol (Sigma)
1 mM ATP (Invitrogen)
20mM DTT (Invitrogen)
10mM spermidine (Sigma)

Incubate at 16°C overnight. Adjust volume to 200u1 with nuclease-free water and perform phenol chloroform pH 7.9 extraction.

Perform ethanol precipitation as follows:

| | |
|---|---|
| DiPETs | 200u1 |
| 3M NaOAC (Ambion) | 20u1 |
| 1M MgCl2 (Ambion) | 4.5u1 |
| Glycoblue (Ambion) | 2.2u1 |
| Abs EtOH (Merck) | 800u1 |

Incubate at -20°C for an hour. Centrifuge at 13,000 RPM for 30mins at 4°C. Wash Ix with 75% ethanol. Resuspend diPETs in 20u1 EB.

After the diPETting reaction, run 1u1 of the DiPET DNA on Agilent Bioanalyzer DNA 500/1000 kit according to the manufacturer's protocol to estimate the concentration and quantity of the DiPETs available. Then, proceed to any preferred method of sequencing (such as '454' sequencing).

### Example 2 - The n-PET embodiment as example

Ligation of two different adaptors. The adaptors may contain source-identifying tags if desired. The adaptors should preferably have one end that is complementary to each other, and ideally, on the other end, the adaptors have sticky ends complementary to sticky ends of the DNA such that ligation will be easy and the adaptors will not ligate to themselves. It is best if the DNA already contains sticky ends, like diPETs. If the DNA is blunt, however, the DNA may be A-tailed with DNA polymerase, and then T-tailed adaptors may be used.

A-tailing (not required if diPETs are used)

| | |
|---|---|
| 10 mm dATP | 0.5 ul |
| ExTaq polymerase | 0.5 ul |
| 10x ExTaq buffer 2 ug | 2.5 ul |
| of sample Nuclease- | 20 ul |
| free Water The total | 1.5 ul |
| volume is 25 ul. | |

Incubate using in a PCR machine with the following program: 72°C for 30 minutes, followed by 4°C forever.

Perform phenol chloroform extract and ethanol precipitation with glycoblue. Resuspend in 12 ul of Elution Buffer.

### Ligation of adaptors

| | | | | | | | | |
|---|---|---|---|---|---|---|---|---|
| DNA (approx 200-1000 ng) | s | a | y | | 5 | | u | l |
| 10x ligase buffer (with | | | 1 | | u | l | | 1 |
| spermidine) T4 DNA ligase (5 | u | l | | t | o | | 1 | 0 |
| U/ul) Nuclease-free Water | | | | | | | u | l |

Incubate at 16°C for 1-2 hours. Ethanol precipitate DNA.

### Phosphorylation of adaptors

| | |
|---|---|
| DNA | say 20 ul |
| 10x T4 PNK buffer | 5 ul |
| 10 mM ATP solution | 5 ul |
| T4 Polynucleotide kinase (3U/ul) | 1 ul |
| Nuclease-free Water | to 50 ul |

Incubate at 37°C for 30 minutes, then heat-inactivate at 70°C for 5 minutes

### Circularization of adaptors

| | |
|---|---|
| Approximately 200 ng DNA | say 50 ul |
| 5x Invitrogen ligation buffer (with PEG) | 20 ul |
| T4 DNA ligase (5U/ul) | 1 ul |
| Nuclease-free Water | to 100 ul |

The DNA should be diluted to a concentration of approximately 2 ng/ul in the final ligation solution - the dilute solution will favour intramolecular ligation.
Incubate at 16°C for 16 hours.

### Exonuclease treatment

| | |
|---|---|
| Adapter-Ligated DNA | say 2 ul |
| Lambda Exonuclease | 1 ul |
| Exonuclease I | 1 ul |
| 10x Lambda Exonuclease buffer | top up to 5 ul if volume of ligated material |
| is less than 5 ul. | |
| Nuclease-free Water | To 50 ul |

Incubate at 37°C for 1 hour.

Perform phenol chloroform extraction and ethanol precipitation with glycoblue. Wash once with 75% ethanol. Resuspend in 12 ul of Elution Buffer. No gel purification is necessary but if desired, if may be performed as follows.

### Gel purification

Gel purify the circularized adaptor-ligated DNA by adding of 0.2 volumes of bromophenol blue gel loading buffer. Load 60 ul per well of a medium sized 2% agarose gel. Electrophorese at 80V for 1.5 hours. Extract circularized mate-pairs with a sharp scalpel, with minimal UV light exposure.

Use Qiagen Gel extraction kit if DNA is larger than 150 bp. Use GeBA-flex Elutatubes if DNA is smaller than 100 bp; perform according to manufacturers' protocols.

Restriction enzyme digest (eg, HindIII, as according to manufacturer's protocols)

### Ligation reaction

| | |
|---|---|
| BamH1/BseR1 cut single PET | 12 ul |
| 10x ligase buffer with spermidine | 1.5 ul |
| T4 DNA ligase (5U/ul) | 1 ul |
| Nuclease-free Water | 0.5 ul |

The total volume is 15 ul. Incubate at 16°C for 16 hours.

Adjust the volume to 200 ul with Nuclease-free Water.

Perform phenol chloroform pH 7.9 extraction with phase lock gel.

Ethanol precipitate with glycoblue

### Quality Control and Quantitation with a PAGE gel

Load approximately 100 ng of sample on a 4-20% gradient PAGE gel, together with Takara Wide Range DNA ladders and Invitrogen Low Mass DNA ladders or other ladders as required for quantitation. Load 1 ul and 2 ul of the Invitrogen Low Mass DNA ladders for more accurate quantitation (FIG. 4).

Repeat to obtain desired length of concatemer.

### PCR modification

If PCR is used for amplification, no exonuclease treatment should be performed. The BD PCR-Select Bacterial Genome Subtraction Kit may be used instead.

### Example 3 - Variation of diPETS or n-PETs having cohesive and non-palindromic, or blunt, ends

This variation is possible by dividing the sample after amplification (Maxiprep or rolling circle amplification or other) into two lots. One lot may be treated with a combination of restriction enzymes, phosphatases and kinases that produce tags with one end that is ligatable, for example a blunt phosphorylated end or a phosphorylated end with an overhang. In this lot, the other end is not ligatable, for example, the other end is a blunt, dephosphorylated end or has either a phosphorylated or dephosphorylated end with an overhang that is not complementary to the first end.

[Num The other lot may be treated with a combination of restriction enzymes, phosphatases and kinases to produce one end that may be ligated, for example a blunt phosphorylated end or a phosphorylated end with an overhang, and another end that cannot be ligated, for example a blunt dephosphorylated end or a phosphorylated or dephosphorylated end with an overhang that is not complementary to the first end, wherein the ligatable end, may be ligated to the ligatable end of the first lot.

Thus, phosphorylated, blunt ends may be ligated to each other, and phsophorylated, cohesive ends complementary to each other may be ligated to each other. For example a digest may produce AA tails in one half, and another digest may produce TT tails in the other half, which can then be mixed in a 1:1 or other suitable ratio to result in dimerization to produce length-controlled diPETs.

### Example 4 - n-PETs with multiples that are not of 2, 4, 8, 16...

First, divide the sample after amplification into any number of aliquots. Then perform the n-pet procedure with these aliquots, repeating any number of times on these aliquots, to end with the production of a blunt, phosphorylated end or a phosphorylated end with an overhang, and another end that cannot be ligated in each aliquot. Ligate suitable aliquots to each other in successive rounds to generate n-PETs with the desired length.

For example, to generate an n-PET consisting of 7 PETs, first separate the sample into 3 aliquots. Then prepare diPETs for two aliquots, and leave one aliquot as it is. With one of the aliquots of diPETs, prepare a 4-PET according to the n-PET method. Finally, combine aliquots: adding the remaining diPET aliquot to the single PET aliquot to form a 3-PET, and then combine the 3-PET with the 4-PET. These steps all require the use of the adapter ligation and selection steps as previously discussed in the n-PET protocol.

### Example 5 - diPETs that have 5' to 3' directionality in different orders

First, divide the sample after amplification into two lots. Then treat one lot with a combination of restriction enzymes, phosphatases and kinases such that the 5' end is a cohesive, non-palindromic end or a blunt end, which may be ligated, while the 3' end cannot be ligated. Treat the other lot with a combination of restriction enzymes, phosphatases, and kinases such that the 3' end is a cohesive, non-palindromic end or a blunt end, which may be ligated, while the 5' end cannot be ligated. Next, combine the two lots and treat with ligase. Fragments from the same lot should not ligate to each other, as their ends are not compatible with each other. However, fragments from different lots should ligate to each other, as their ends have been selected to be cohesive, and thus compatible, with each other.

### Example 6 - Chromatin Interaction Precipitation diPETting (ChIP-PET).

ChIP-PET is a method to identify DNA regions that interact with proteins such as those found in chromatin structures. This variation requires a different vector, pGIS3h (FIG. 5B, SEQ ⁻ID NO:15). When cut, this produces diPETs of about 88 base pairs and 4 residues (there are 2 CG residues on either end). There are no AA tails. In contrast, the other variations of the present invention uses pGIS4a2 (FIG. 5A; SEQ ID NO:14). This produces diPETs of a total size of 80 base pairs and 4 residues (there are 2 AA residues on either end).

### References

Adams, M., et al., 1991, Science, 252, 1651-1656
Brenner S, et al., 2000, Nature Biotechnology, 18, 630-634
Dunn et al (2002) Genome Research 12(11):1756-1765
Jongeneel et al., 2003, Proc. Natl. Acad. Sci. USA 100: 4702-4705
Li and Chandrasegaran, Proc. Nat. Acad. Sciences USA 90:2764-8, 1993
Margulies M. et al., Nature, 31 July 2005.
Strausberg, R.L., et al., 1999, Science, 286: 455-457
Velculescu, V. E., et al., 1995, Science, 270, 484-487
US 4,766,072
US 20050255501
US 20050059022

## Claims

1. A method of length-controlled concatenating nucleotide fragments, the method comprising:
(a) providing at least two nucleotide fragments, wherein each fragment has one ligatable end and one non-ligatable end;
(b) allowing the two fragments to ligate at the ligatable ends to form at least one oligonucleotide comprising at least two concatenated nucleotide fragments,
(c) further treating the at least one oligonucleotide from step (b) to produce at least one oligonucleotide with one ligatable end and one non-ligatable end; and
(d) allowing the oligonucleotide to ligate with a further oligonucleotide comprising one ligatable end and one non-ligatable end to form an oligonucleotide comprising more than two concatenated nucleotide fragments.

2. The method according to claim 1, wherein the ligatable end of each fragment is a palindromic cohesive end.

3. The method according to claim 1 or 2, wherein the ligatable end and/or the non-ligatable end is located in at least one adaptor.

4. The method according to any one of the preceding claims, wherein the steps (c) and (d) are repeated one or more times.

5. The method according to any one of claims 1 to 3, further comprising treating the at least one oligonucleotide to produce at least one oligonucleotide with two ligatable ends and allowing the oligonucleotide to self-circularize at the ligatable ends;
selecting the at least one circularized oligonucleotide and/or amplifying the oligonucleotide;
treating the at least one circularized and/or amplified oligonucleotide to produce at least one oligonucleotide having one ligatable end and one non-ligatable end, and allowing the oligonucleotide to ligate with at least a further oligonucleotide at their ligatable ends to form at least two concatenated oligonucleotides.

6. A method of length-controlled concatenating nucleotide fragments, the method comprising the steps of:
(a) providing at least one oligonucleotide comprising at least one nucleotide fragment, wherein the oligonucleotide has ligatable ends;
(b) allowing the at least one oligonucleotide to self-circularize at its ligatable ends;
(c) selecting and optionally amplifying the at least one self-circularized oligonucleotide;
(d) treating the oligonucleotides with at least one restriction enzyme to obtain at least one oligonucleotide with one ligatable end and one non- ligatable end; and
(e) concatenating two oligonucleotides at the ligatable ends to form a concatenated oligonucleotide comprising two nucleotide fragments.

7. The method according to any one of claims 1 to 6, wherein the nucleotide fragment(s) comprises at least one ditag, the ditag comprising at least one first tag comprising a 5' terminus and at least one second tag comprising a 3' terminus of a polynucleotide.

8. The method according to any one of claims 5 to 7, wherein the ligatable ends are palindromic cohesive ends.

9. The method according to any one of claims 5 to 8, wherein the ligatable end and/or the non-ligatable end is located in at least one adaptor

10. The method according to claim 5, wherein the steps are repeated one or more times to obtain concatemers having the desired lengths and/or number of oligonucleotides or nucleotide fragments.

11. The method according to any one of claims 5 to 10, wherein the amplification is by rolling circle amplification, polymerase chain reaction or bacterial amplification.

12. The method according to any one of the preceding claims, wherein the two nucleotide fragments are of different sizes.

13. The method according to any one of claims 1 to 12, the method further comprising sequencing the concatenated oligonucleotide.

## Patentansprüche

1. Verfahren zum längenkontrollierten Verknüpfen von Nukleotidfragmenten, wobei das Verfahren umfasst:
(a) Bereitstellen von wenigstens zwei Nukleotidfragmenten, wobei jedes Fragment ein ligierbares Ende und ein nicht-ligierbares Ende aufweist;
(b) Erlauben, dass die zwei Fragmente an den ligierbaren Enden ligieren, um wenigstens ein Oligonukleotid auszubilden, das wenigstens zwei verknüpfte Nukleotidfragmente umfasst,
(c) weiter Behandeln des wenigstens einen Oligonukleotids aus Schritt (b), um wenigstens ein Oligonukleotid mit einem ligierbaren Ende und einem nicht-ligierbaren Ende herzustellen; und
(d) Erlauben, dass das Oligonukleotid mit einem weiteren Oligonukleotid ligiert, das ein ligierbares Ende und ein nicht-ligierbares Ende umfasst, um ein Oligonukleotid auszubilden, das mehr als zwei verknüpfte Nukleotidfragmente umfasst.

2. Verfahren nach Anspruch 1, wobei das ligierbare Ende eines jeden Fragmentes ein kohäsives Palindromende ist.

3. Verfahren nach Anspruch 1 oder 2, wobei sich das ligierbare Ende und/oder das nichtligierbare Ende in wenigstens einem Adapter befindet.

4. Verfahren nach einem der vorhergehenden Ansprüche, wobei die Schritte (c) und (d) ein oder mehrmals wiederholt werden.

5. Verfahren nach einem der Ansprüche 1 bis 3, weiter umfassend Behandeln des wenigstens einen Oligonukleotids, um wenigstens ein Oligonukleotid zu erhalten mit zwei ligierbaren Enden, und Erlauben, dass das Oligonukleotid an den ligierbaren Enden mit sich selbst zirkularisiert;
Auswählen des wenigstens einen zirkularisierten Oligonukleotids und/oder Amplifizieren des Oligonukleotids;
Behandeln des wenigstens einen zirkularisierten und/oder amplifizierten Oligonukleotids, um wenigstens ein Oligonukleotid mit wenigstens einem ligierbaren Ende und einem nichtligierbaren Ende herzustellen, und Erlauben, dass das Oligonukleotid mit wenigstens einem weiteren Oligonukleotid an ihren ligierbaren Enden ligiert, um wenigstens zwei verknüpfte Oligonukleotide auszubilden.

6. Verfahren zum längenkontrollierten Verknüpfen von Nukleotidfragmenten, wobei das Verfahren die Schritte umfasst:
(a) Bereitstellen von wenigstens einem Oligonukleotid umfassend wenigstens ein Nukleotidfragment, wobei das Oligonukleotid ligierbare Enden aufweist;
(b) Erlauben, dass das wenigstens eine Oligonukleotid an seinen ligierbaren Enden mit sich selbst zirkularisiert;
(c) Auswählen und optional Amplifizieren des wenigstens einen selbstzirkularisierten Oligonukleotids;
(d) Behandeln der Oligonukleotide mit wenigstens einem Restriktionsenzym, um wenigstens ein Oligonukleotid mit einem ligierbaren Ende und einem nicht-ligierbaren Ende zu erhalten; und
(e) Verknüpfen von zwei Oligonukleotiden an den ligierbaren Enden, um ein verknüpftes Oligonukleotid umfassend zwei Nukleotidfragmente zu erhalten.

7. Verfahren nach einem der Ansprüche 1 bis 6, wobei das/die Nukleotidfragment(e) wenigstens eine Markierung mit gepaartem Ende umfasst/umfassen, wobei die Markierung mit gepaartem Ende wenigstens eine erste Markierung umfasst, die einen 5'-Terminus eines Polynukleotids umfasst, und wenigstens eine zweite Markierung, die einen 3'-Terminus eines Polynukleotids umfasst.

8. Verfahren nach einem der Ansprüche 5 bis 7, wobei die ligierbaren Enden kohäsive Palindromenden sind.

9. Verfahren nach einem der Ansprüche 5 bis 8, wobei das ligierbare Ende und/oder nicht-ligierbare Ende sich in wenigstens einem Adapter befindet/befinden.

10. Verfahren nach Anspruch 5, wobei die Schritte einmal oder mehrfach wiederholt werden, um Konkatemere mit den erwünschten Längen und/oder der erwünschten Anzahl von Oligonukleotiden oder Nukleotidfragmenten zu erhalten.

11. Verfahren nach einem der Ansprüche 5 bis 10, wobei die Amplifikation eine Rolling Circle-Amplifikation, Polymerasekettenreaktion oder bakterielle Amplifikation ist.

12. Verfahren nach einem der vorhergehenden Ansprüche, wobei die zwei Nukleotidfragmente von unterschiedlicher Größe sind.

13. Verfahren nach einem der Ansprüche 1 bis 12, wobei das Verfahren weiter Sequenzieren des verknüpften Oligonukleotids umfasst.

## Revendications

1. Procédé de concaténation régulée par la longueur de fragments nucléotidiques, le procédé comprenant le fait :
(a) de fournir au moins deux fragments nucléotidiques, où chaque fragment a une extrémité pouvant être ligaturée et une extrémité ne pouvant pas être ligaturée ;
(b) de permettre la ligature des deux fragments au niveau des extrémités pouvant être ligaturées pour former au moins un oligonucléotide comprenant au moins deux fragments nucléotidiques concaténés,
(c) de traiter en outre l'au moins un oligonucléotide de l'étape (b) pour produire au moins un oligonucléotide avec une extrémité pouvant être ligaturée et une extrémité ne pouvant pas être ligaturée ; et
(d) de permettre la ligature de l'oligonucléotide avec un oligonucléotide supplémentaire comprenant une extrémité pouvant être ligaturée et une extrémité ne pouvant pas être ligaturée pour former un oligonucléotide comprenant plus de deux fragments nucléotidiques concaténés.

2. Procédé selon la revendication 1, dans lequel l'extrémité pouvant être ligaturée de chaque fragment est une extrémité cohésive palindromique.

3. Procédé selon la revendication 1 ou 2, dans lequel l'extrémité pouvant être ligaturée et/ou l'extrémité ne pouvant pas être ligaturée est/sont située(s) dans au moins un adaptateur.

4. Procédé selon l'une quelconque des revendications précédentes, dans lequel les étapes (c) et (d) sont répétées une ou plusieurs fois.

5. Procédé selon l'une quelconque des revendications 1 à 3, comprenant en outre le fait
de traiter l'au moins un oligonucléotide pour produire au moins un oligonucléotide avec deux extrémités pouvant être ligaturées et de permettre à l'oligonucléotide de s'auto-circulariser au niveau des extrémités pouvant être ligaturées ;
de sélectionner l'au moins un oligonucléotide circularisé et/ou d'amplifier l'oligonucléotide ;
de traiter l'au moins un oligonucléotide circularisé et/ou amplifié pour produire au moins un oligonucléotide ayant une extrémité pouvant être ligaturée et une extrémité ne pouvant pas être ligaturée, et
de permettre la ligature de l'oligonucléotide avec au moins un oligonucléotide supplémentaire au niveau de leurs extrémités pouvant être ligaturées pour former au moins deux oligonucléotides concaténés.

6. Procédé de concaténation régulée par la longueur de fragments nucléotidiques, le procédé comprenant les étapes qui consistent :
(a) à fournir au moins un oligonucléotide comprenant au moins un fragment nucléotidique, où l'oligonucléotide a des extrémités pouvant être ligaturées ;
(b) à permettre à l'au moins un oligonucléotide de s'auto-circulariser au niveau de ses extrémités pouvant être ligaturées ;
(c) à sélectionner et à amplifier facultativement l'au moins un oligonucléotide auto-circularisé ;
(d) à traiter les oligonucléotides avec au moins une enzyme de restriction pour obtenir au moins un oligonucléotide avec une extrémité pouvant être ligaturée et une extrémité ne pouvant pas être ligaturée ; et
(e) à concaténer deux oligonucléotides au niveau des extrémités pouvant être ligaturées pour former un oligonucléotide concaténé comprenant deux fragments nucléotidiques.

7. Procédé selon l'une quelconque des revendications 1 à 6, dans lequel le/les fragment(s) nucléotidique(s) comprend/comprennent au moins une double étiquette, la double étiquette comprenant au moins une première étiquette comprenant une extrémité 5' terminale et au moins une deuxième étiquette comprenant une extrémité 3' terminale d'un polynucléotide.

8. Procédé selon l'une quelconque des revendications 5 à 7, dans lequel les extrémités pouvant être ligaturées sont des extrémités cohésives palindromiques.

9. Procédé selon l'une quelconque des revendications 5 à 8, dans lequel l'extrémité pouvant être ligaturée et/ou l'extrémité ne pouvant pas être ligaturée est/sont située(s) dans au moins un adaptateur.

10. Procédé selon la revendication 5, dans lequel les étapes sont répétées une ou plusieurs fois pour obtenir des concatémères ayant les longueurs souhaitées et/ou le nombre souhaité d'oligonucléotides ou de fragments nucléotidiques.

11. Procédé selon l'une quelconque des revendications 5 à 10, dans lequel l'amplification est effectuée par une amplification par cercle roulant, une réaction en chaîne par polymérase ou une amplification bactérienne.

12. Procédé selon l'une quelconque des revendications précédentes, dans lequel les deux fragments nucléotidiques sont de tailles différentes.

13. Procédé selon l'une quelconque des revendications 1 à 12, le procédé comprenant en outre le séquençage de l'oligonucléotide concaténé.


### SEQUENCE LISTING

<110> Agency for Science, Technology and Research
<120> NUCLEIC ACID CONCATENATION
<130> FP3677
<160> 21
<170> PatentIn version 3.4
<210> 1
   <211> 33
   <212> DNA
   <213> ARTIFICIAL
<220>
   <223> GsuI-oligo dT primer
<220>
   <221> misc_feature
   <222> (33)..(33)
   <223> n is a, c, g, or t
<400> 1
   gagctagttc tggagttttt tttttttttt tvn 33
<210> 2
   <211> 34
   <212> DNA
   <213> ARTIFICIAL
<220>
   <223> GIS-(N)6 adapter upper strand
<220>
   <221> misc_feature
   <222> (29)..(34)
   <223> n is a, c, g, or t
<400> 2
   ctaaactcga ggcggccgcg gatccgacnn nnnn 34
<210> 3
   <211> 26
   <212> DNA
   <213> ARTIFICIAL
<220>
   <223> GIS-(N)6 adapter lower strand
<400> 3
   gtcggatccg cggccgcctc gagttt 26
<210> 4
   <211> 34
   <212> DNA
   <213> ARTIFICIAL
<220>
   <223> GIS-(N)5 adapter upper strand
<220>
   <221> misc_feature
   <222> (30)..(34)
   <223> n is a, c, g, or t
<400> 4
   ctaaactcga ggcggccgcg gatccgacgn nnnn 34
<210> 5
   <211> 26
   <212> DNA
   <213> ARTIFICIAL
<220>
   <223> GIS-(N)5 adapter lower strand
<400> 5
   gtcggatccg cggccgcctc gagttt 26
<210> 6
   <211> 12
   <212> DNA
   <213> ARTIFICIAL
<220>
   <223> palindromic upper strand
<400> 6
   gtcggatccg ac 12
<210> 7
   <211> 12
   <212> DNA
   <213> ARTIFICIAL
<220>
   <223> palindromic lower strand
<400> 7
   gtcggatccg ac 12
<210> 8
   <211> 40
   <212> DNA
   <213> ARTIFICIAL
<220>
   <223> n-PET TT-tailed adaptor (PMR 011) - upper strand
<400> 8
   gcttgtaagc tactcctcga tgtgctgcaa ggcgattaag 40
<210> 9
   <211> 42
   <212> DNA
   <213> ARTIFICIAL
<220>
   <223> N-PET TT-tailed adaptor (PMR 011) - lower strand
<400> 9
   ttcgaacatt cgatgaggag ctacacgacg ttccgctaat tc 42
<210> 10
   <211> 41
   <212> DNA
   <213> ARTIFICIAL
<220>
   <223> n-PET TT-tailed adaptor (PMR 012) - upper strand
<400> 10
   gcttgtaagc tactcctcag cggataacaa tttcacacag g 41
<210> 11
   <211> 43
   <212> DNA
   <213> ARTIFICIAL
<220>
   <223> n-PET TT-tailed adaptor (PMR 012) - lower strand
<400> 11
   ttcgaacatt cgatgaggag tcgcctattg ttaaagtgtg tcc 43
<210> 12
   <211> 22
   <212> DNA
   <213> ARTIFICIAL
<220>
   <223> PMR011
<400> 12
   gatgtgctgc aaggcgatta ag 22
<210> 13
   <211> 23
   <212> DNA
   <213> ARTIFICIAL
<220>
   <223> PMR012
<400> 13
   agcggataac aatttcacac agg 23
<210> 14
   <211> 3531
   <212> DNA
   <213> ARTIFICIAL
<220>
   <223> pGIS4a2 - sense (FIG. 5A)
<400> 14
   gggcgaattc gatatcgcgg ccgcgcctgg ataaagtcag cagcttccac gccagcttca    60
   cacaaaaagt gactgacggt agcggcgcgg cggtgcagga aggtcagggc gatctgtggg   120
   tgaaacgtcc aaacttattc aactggcata tgacacaacc tgatgaaagc attctggttt   180
   ctgacggtaa aacactgtgg ttctataacc cgttcgttga gcaagctacg gcaacctggc   240
   tgaaagatgc caccggtaat acgccgttta tgctgattgc ccgcaaccag tccagcgact   300
   ggcagcagta caatatcaaa cagaatggcg atgactttgt cctgacgccg aaagccagca   360
   atggcaatct gaagcagttc accattaacg tgggacgtga tggcacaatc catcagttta   420
   gcgcggtgga gcaggacgat cagcgcagca gttatcaact gaaatcccag caaaatgggg   480
   ctgtggatgc agcgaaattt accttcaccc cgccgcaagg cgtcacggta gatgatcaac   540
   gtaagtagag gcacctgagt gagcaatctg tcgctcgatt tttcggataa tacttttcaa   600
   cctctggccg cgcgtatgcg gccagaaaat ttagcacagt atatcggcca gcaacatttg   660
   ctggctgcgg ggaagccgtt gccgcgcgct atcgaagccg ggcatttaca ttctatgatc   720
   ctctgggggc cgccgggtac cggcaaaaca actctcgctg aagtgattgc ccgctatgcg   780
   aacgctgatg tggaacgtat ttctgccgta agtcggatcc tcctcgtcga cctgcaggca   840
   tgcaagcttg agtattctat agtgtcacct aaatagcttg gcgtaatcat ggtcatagct   900
   gtttcctgtg tgaaattgtt atccgctcac aattccacac aacatacgag ccggaagcat   960
   aaagtgtaaa gcctggggtg cctaatgagt gagctaactc acattaattg cgttgcgctc  1020
   actgcccgct ttccagtcgg gaaacctgtc gtgccagctg cattaatgaa tcggccaacg  1080
   cgcggggaga ggcggtttgc gtattgggcg ctcttccgct tcctcgctca ctgactcgct  1140
   gcgctcggtc gttcggctgc ggcgagcggt atcagctcac tcaaaggcgg taatacggtt  1200
   atccacagaa tcaggggata acgcaggaaa gaacatgtga gcaaaaggcc agcaaaaggc  1260
   caggaaccgt aaaaaggccg cgttgctggc gtttttcgat aggctccgcc cccctgacga   1320
   gcatcacaaa aatcgacgct caagtcagag gtggcgaaac ccgacaggac tataaagata   1380
   ccaggcgttt ccccctggaa gctccctcgt gcgctctcct gtaccgaccc tgccgcttac   1440
   cggatacctg tccgcctttc tcccttcggg aagcgtggcg ctttctcata gctcacgctg   1500
   taggtatctc agttcggtgt aggtcgttcg ctccaagctg ggctgtgtgc acgaaccccc   1560
   cgttcagccc gaccgctgcg ccttatccgg taactatcgt cttgagacca acccggtaag   1620
   acacgactta tcgccactgg cagcagccac tggtaacagg attagcagag cgaggtatgt   1680
   aggcggtgct acagagttct tgaagtggtg gcctaactac ggctacacta gaaggacagt   1740
   atttggtatc tgcgctctgc tgaagccagt taccttcgga aaaagagttg gtagctcttg   1800
   atccggcaaa caaaccaccg ctggtagcgg tggttttttt gtttgcaagc agcagattac   1860
   gcgcagaaaa aaaggatctc aagaagatcc tttgatcttt tctacggggt ctgacgctca   1920
   gtggaacgaa aactcacgtt aagggatttt ggtcatgaga ttatcaaaaa ggatcttcac   1980
   ctagatcctt ttaaattaaa aatgaagttt taaatcaatc taaagtatat atgagtaaac   2040
   ttggtctgac agttaccaat gcttaatcag tgaggcacct atctcagcga tctgtctatt   2100
   tcgttcatcc atagttgcct gactccccgt cgtgtagata actacgatac gggagggctt   2160
   accatctggc cccagtgctg caatgatacc gcgagaccca cgctcaccgg ctccagattt   2220
   atcagcaata aaccagccag ccggaagggc cgagcgcaga agtggtcctg caactttatc   2280
   cgcctccatc cagtctatta attgttgccg ggaagctaga gtaagtagtt cgccagttaa   2340
   tagtttgcgc aacgttgttg gcattgctac aggcatcgtg gtgtcacgct cgtcgtttgg   2400
   tatggcttca ttcagctccg gttcccaacg atcaaggcga gttacatgat cccccatgtt   2460
   gtgcaaaaaa gcggttagct ccttcggtcc tccgatcgtt gtcagaagta agttggccgc   2520
   agtgttatca ctcatggtta tggcagcact gcataattct cttactgtca tgccatccgt   2580
   aagatgcttt tctgtgactg gtgagtactc aaccaagtca ttctgagaat agtgtatgcg   2640
   gcgaccgagt tgctcttgcc cggcgtcaat acgggataat accgcgccac atagcagaac   2700
   tttaaaagtg ctcatcattg gaaaacgttc ttcggggcga aaactctcaa ggatcttacc   2760
   gctgttgaga tccagttcga tgtaacccac tcgtgcaccc aactgatctt cagcatcttt   2820
   tactttcacc agcgtttctg ggtgagcaaa aacaggaagg caaaatgccg caaaaaaggg   2880
   aataagggcg acacggaaat gttgaatact catactcttc ctttttcaat attattgaag   2940
   catttatcag ggttattgtc tcatgagcgg atacatattt gaatgtattt agaaaaataa   3000
   acaaataggg gttccgcgca catttccccg aaaagtgcca cctgacgtct aagaaaccat   3060
   tattatcatg acattaacct ataaaaatag gcgtatcacg aggccctttc gtctcgcgcg   3120
   tttcggtgat gacggtgaaa acctctgaca catgcagctc ccggagacgg tcacagcttg   3180
   tctgtaagcg gatgccggga gcagacaagc ccgtcagggc gcgtcagcgg gtgttggcgg   3240
   gtgtcggggc tggcttaact atgcggcatc agagcagatt gtactgagag tgcaccatat   3300
   gcggtgtgaa ataccgcaca gatgcgtaag gagaaaatac cgcatcaggc gccattcgcc   3360
   attcaggctg cgcaactgtt gggaagggcg atcggtgcgg gcctcttcgc tattacgcca   3420
   gctggcgaaa gggggatgtg ctgcaaggcg attaagttgg gtaacgccag ggttttccca   3480
   gtcacgacgt tgtaaaacga cggccagtga attgtaatac gactcactat a            3531
<210> 15
   <211> 3531
   <212> DNA
   <213> ARTIFICIAL
<220>
   <223> pGIS4a2 - antisense (FIG. 5A)
<400> 15
   tatagtgagt cgtattacaa ttcactggcc gtcgttttac aacgtcgtga ctgggaaaac     60
   cctggcgtta cccaacttaa tcgccttgca gcacatcccc ctttcgccag ctggcgtaat    120
   agcgaagagg cccgcaccga tcgcccttcc caacagttgc gcagcctgaa tggcgaatgg    180
   cgcctgatgc ggtattttct ccttacgcat ctgtgcggta tttcacaccg catatggtgc    240
   actctcagta caatctgctc tgatgccgca tagttaagcc agccccgaca cccgccaaca    300
   cccgctgacg cgccctgacg ggcttgtctg ctcccggcat ccgcttacag acaagctgtg    360
   accgtctccg ggagctgcat gtgtcagagg ttttcaccgt catcaccgaa acgcgcgaga    420
   cgaaagggcc tcgtgatacg cctattttta taggttaatg tcatgataat aatggtttct    480
   tagacgtcag gtggcacttt tcggggaaat gtgcgcggaa cccctatttg tttatttttc    540
   taaatacatt caaatatgta tccgctcatg agacaataac cctgataaat gcttcaataa    600
   tattgaaaaa ggaagagtat gagtattcaa catttccgtg tcgcccttat tccctttttt    660
   gcggcatttt gccttcctgt ttttgctcac ccagaaacgc tggtgaaagt aaaagatgct    720
   gaagatcagt tgggtgcacg agtgggttac atcgaactgg atctcaacag cggtaagatc    780
   cttgagagtt ttcgccccga agaacgtttt ccaatgatga gcacttttaa agttctgcta    840
   tgtggcgcgg tattatcccg tattgacgcc gggcaagagc aactcggtcg ccgcatacac    900
   tattctcaga atgacttggt tgagtactca ccagtcacag aaaagcatct tacggatggc    960
   atgacagtaa gagaattatg cagtgctgcc ataaccatga gtgataacac tgcggccaac 1020
   ttacttctga caacgatcgg aggaccgaag gagctaaccg cttttttgca caacatgggg 1080
   gatcatgtaa ctcgccttga tcgttgggaa ccggagctga atgaagccat accaaacgac 1140
   gagcgtgaca ccacgatgcc tgtagcaatg ccaacaacgt tgcgcaaact attaactggc 1200
   gaactactta ctctagcttc ccggcaacaa ttaatagact ggatggaggc ggataaagtt 1260
   gcaggaccac ttctgcgctc ggcccttccg gctggctggt ttattgctga taaatctgga 1320
   gccggtgagc gtgggtctcg cggtatcatt gcagcactgg ggccagatgg taagccctcc 1380
   cgtatcgtag ttatctacac gacggggagt caggcaacta tggatgaacg aaatagacag 1440
   atcgctgaga taggtgcctc actgattaag cattggtaac tgtcagacca agtttactca 1500
   tatatacttt agattgattt aaaacttcat ttttaattta aaaggatcta ggtgaagatc 1560
   ctttttgata atctcatgac caaaatccct taacgtgagt tttcgttcca ctgagcgtca 1620
   gaccccgtag aaaagatcaa aggatcttct tgagatcctt tttttctgcg cgtaatctgc 1680
   tgcttgcaaa caaaaaaacc accgctacca gcggtggttt gtttgccgga tcaagagcta 1740
   ccaactcttt ttccgaaggt aactggcttc agcagagcgc agataccaaa tactgtcctt 1800
   ctagtgtagc cgtagttagg ccaccacttc aagaactctg tagcaccgcc tacatacctc 1860
   gctctgctaa tcctgttacc agtggctgct gccagtggcg ataagtcgtg tcttaccggg 1920
   ttggactcaa gacgatagtt accggataag gcgcagcggt cgggctgaac ggggggttcg 1980
   tgcacacagc ccagcttgga gcgaacgacc tacaccgaac tgagatacct acagcgtgag 2040
   ctatgagaaa gcgccacgct tcccgaaggg agaaaggcgg acaggtatcc ggtaagcggc 2100
   agggtcggaa caggagagcg cacgagggag cttccagggg gaaacgcctg gtatctttat 2160
   agtcctgtcg ggtttcgcca cctctgactt gagcgtcgat ttttgtgatg ctcgtcaggg 2220
   gggcggagcc tatggaaaaa cgccagcaac gcggcctttt tacggttcct ggccttttgc 2280
   tggccttttg ctcacatgtt ctttcctgcg ttatcccctg attctgtgga taaccgtatt 2340
   accgcctttg agtgagctga taccgctcgc cgcagccgaa cgaccgagcg cagcgagtca 2400
   gtgagcgagg aagcggaaga gcgcccaata cgcaaaccgc ctctccccgc gcgttggccg 2460
   attcattaat gcagctggca cgacaggttt cccgactgga aagcgggcag tgagcgcaac 2520
   gcaattaatg tgagttagct cactcattag gcaccccagg ctttacactt tatgcttccg 2580
   gctcgtatgt tgtgtggaat tgtgagcgga taacaatttc acacaggaaa cagctatgac 2640
   catgattacg ccaagctatt taggtgacac tatagaatac tcaagcttgc atgcctgcag 2700
   gtcgacgagg aggatccgac ttacggcaga aatacgttcc acatcagcgt tcgcatagcg   2760
   ggcaatcact tcagcgagag ttgttttgcc ggtacccggc ggcccccaga ggatcataga   2820
   atgtaaatgc ccggcttcga tagcgcgcgg caacggcttc cccgcagcca gcaaatgttg   2880
   ctggccgata tactgtgcta aattttctgg ccgcatacgc gcggccagag gttgaaaagt   2940
   attatccgaa aaatcgagcg acagattgct cactcaggtg cctctactta cgttgatcat   3000
   ctaccgtgac gccttgcggc ggggtgaagg taaatttcgc tgcatccaca gccccatttt   3060
   gctgggattt cagttgataa ctgctgcgct gatcgtcctg ctccaccgcg ctaaactgat   3120
   ggattgtgcc atcacgtccc acgttaatgg tgaactgctt cagattgcca ttgctggctt   3180
   tcggcgtcag gacaaagtca tcgccattct gtttgatatt gtactgctgc cagtcgctgg   3240
   actggttgcg ggcaatcagc ataaacggcg tattaccggt ggcatctttc agccaggttg   3300
   ccgtagcttg ctcaacgaac gggttataga accacagtgt tttaccgtca gaaaccagaa   3360
   tgctttcatc aggttgtgtc atatgccagt tgaataagtt tggacgtttc acccacagat   3420
   cgccctgacc ttcctgcacc gccgcgccgc taccgtcagt cactttttgt gtgaagctgg   3480
   cgtggaagct gctgacttta tccaggcgcg gccgcgatat cgaattcgcc c            3531
<210> 16
   <211> 2765
   <212> DNA
   <213> ARTIFICIAL
<220>
   <223> pGIS3h - sense (FIG. 5B)
<400> 16
   gggcgaattc gatatcgcgg ccgcgaggat tatggatccg actgcagtcg gatccatact     60
   cctcattgca ggcatgcaag cttgagtatt ctatagtgtc acctaaatag cttggcgtaa    120
   tcatggtcat agctgtttcc tgtgtgaaat tgttatccgc tcacaattcc acacaacata    180
   cgagccggaa gcataaagtg taaagcctgg ggtgcctaat gagtgagcta actcacatta    240
   attgcgttgc gctcactgcc cgctttccag tcgggaaacc tgtcgtgcca gctgcattaa    300
   tgaatcggcc aacgcgcggg gagaggcggt ttgcgtattg ggcgctcttc cgcttcctcg    360
   ctcactgact cgctgcgctc ggtcgttcgg ctgcggcgag cggtatcagc tcactcaaag    420
   gcggtaatac ggttatccac agaatcaggg gataacgcag gaaagaacat gtgagcaaaa    480
   ggccagcaaa aggccaggaa ccgtaaaaag gccgcgttgc tggcgttttt cgataggctc    540
   cgcccccctg acgagcatca caaaaatcga cgctcaagtc agaggtggcg aaacccgaca    600
   ggactataaa gataccaggc gtttccccct ggaagctccc tcgtgcgctc tcctgtaccg    660
   accctgccgc ttaccggata cctgtccgcc tttctccctt cgggaagcgt ggcgctttct     720
   catagctcac gctgtaggta tctcagttcg gtgtaggtcg ttcgctccaa gctgggctgt     780
   gtgcacgaac cccccgttca gcccgaccgc tgcgccttat ccggtaacta tcgtcttgag     840
   accaacccgg taagacacga cttatcgcca ctggcagcag ccactggtaa caggattagc     900
   agagcgaggt atgtaggcgg tgctacagag ttcttgaagt ggtggcctaa ctacggctac     960
   actagaagga cagtatttgg tatctgcgct ctgctgaagc cagttacctt cggaaaaaga    1020
   gttggtagct cttgatccgg caaacaaacc accgctggta gcggtggttt ttttgtttgc    1080
   aagcagcaga ttacgcgcag aaaaaaagga tctcaagaag atcctttgat cttttctacg    1140
   gggtctgacg ctcagtggaa cgaaaactca cgttaaggga ttttggtcat gagattatca    1200
   aaaaggatct tcacctagat ccttttaaat taaaaatgaa gttttaaatc aatctaaagt    1260
   atatatgagt aaacttggtc tgacagttac caatgcttaa tcagtgaggc acctatctca    1320
   gcgatctgtc tatttcgttc atccatagtt gcctgactcc ccgtcgtgta gataactacg    1380
   atacgggagg gcttaccatc tggccccagt gctgcaatga taccgcgaga cccacgctca    1440
   ccggctccag atttatcagc aataaaccag ccagccggaa gggccgagcg cagaagtggt    1500
   cctgcaactt tatccgcctc catccagtct attaattgtt gccgggaagc tagagtaagt    1560
   agttcgccag ttaatagttt gcgcaacgtt gttggcattg ctacaggcat cgtggtgtca    1620
   cgctcgtcgt ttggtatggc ttcattcagc tccggttccc aacgatcaag gcgagttaca    1680
   tgatccccca tgttgtgcaa aaaagcggtt agctccttcg gtcctccgat cgttgtcaga    1740
   agtaagttgg ccgcagtgtt atcactcatg gttatggcag cactgcataa ttctcttact    1800
   gtcatgccat ccgtaagatg cttttctgtg actggtgagt actcaaccaa gtcattctga    1860
   gaatagtgta tgcggcgacc gagttgctct tgcccggcgt caatacggga taataccgcg    1920
   ccacatagca gaactttaaa agtgctcatc attggaaaac gttcttcggg gcgaaaactc    1980
   tcaaggatct taccgctgtt gagatccagt tcgatgtaac ccactcgtgc acccaactga    2040
   tcttcagcat cttttacttt caccagcgtt tctgggtgag caaaaacagg aaggcaaaat    2100
   gccgcaaaaa agggaataag ggcgacacgg aaatgttgaa tactcatact cttccttttt    2160
   caatattatt gaagcattta tcagggttat tgtctcatga gcggatacat atttgaatgt    2220
   atttagaaaa ataaacaaat aggggttccg cgcacatttc cccgaaaagt gccacctgac    2280
   gtctaagaaa ccattattat catgacatta acctataaaa ataggcgtat cacgaggccc    2340
   tttcgtctcg cgcgtttcgg tgatgacggt gaaaacctct gacacatgca gctcccggag    2400
   acggtcacag cttgtctgta agcggatgcc gggagcagac aagcccgtca gggcgcgtca 2460
   gcgggtgttg gcgggtgtcg gggctggctt aactatgcgg catcagagca gattgtactg 2520
   agagtgcacc atatgcggtg tgaaataccg cacagatgcg taaggagaaa ataccgcatc 2580
   aggcgccatt cgccattcag gctgcgcaac tgttgggaag ggcgatcggt gcgggcctct 2640
   tcgctattac gccagctggc gaaaggggga tgtgctgcaa ggcgattaag ttgggtaacg 2700
   ccagggtttt cccagtcacg acgttgtaaa acgacggcca gtgaattgta atacgactca 2760
   ctata 2765
<210> 17
   <211> 2765
   <212> DNA
   <213> ARTIFICIAL
<220>
   <223> pGIS3h - antisense (FIG. 15B)
<400> 17
   tatagtgagt cgtattacaa ttcactggcc gtcgttttac aacgtcgtga ctgggaaaac 60
   cctggcgtta cccaacttaa tcgccttgca gcacatcccc ctttcgccag ctggcgtaat 120
   agcgaagagg cccgcaccga tcgcccttcc caacagttgc gcagcctgaa tggcgaatgg 180
   cgcctgatgc ggtattttct ccttacgcat ctgtgcggta tttcacaccg catatggtgc 240
   actctcagta caatctgctc tgatgccgca tagttaagcc agccccgaca cccgccaaca 300
   cccgctgacg cgccctgacg ggcttgtctg ctcccggcat ccgcttacag acaagctgtg 360
   accgtctccg ggagctgcat gtgtcagagg ttttcaccgt catcaccgaa acgcgcgaga 420
   cgaaagggcc tcgtgatacg cctattttta taggttaatg tcatgataat aatggtttct 480
   tagacgtcag gtggcacttt tcggggaaat gtgcgcggaa cccctatttg tttatttttc 540
   taaatacatt caaatatgta tccgctcatg agacaataac cctgataaat gcttcaataa 600
   tattgaaaaa ggaagagtat gagtattcaa catttccgtg tcgcccttat tccctttttt 660
   gcggcatttt gccttcctgt ttttgctcac ccagaaacgc tggtgaaagt aaaagatgct 720
   gaagatcagt tgggtgcacg agtgggttac atcgaactgg atctcaacag cggtaagatc 780
   cttgagagtt ttcgccccga agaacgtttt ccaatgatga gcacttttaa agttctgcta 840
   tgtggcgcgg tattatcccg tattgacgcc gggcaagagc aactcggtcg ccgcatacac 900
   tattctcaga atgacttggt tgagtactca ccagtcacag aaaagcatct tacggatggc 960
   atgacagtaa gagaattatg cagtgctgcc ataaccatga gtgataacac tgcggccaac 1020
   ttacttctga caacgatcgg aggaccgaag gagctaaccg cttttttgca caacatgggg 1080
   gatcatgtaa ctcgccttga tcgttgggaa ccggagctga atgaagccat accaaacgac 1140
   gagcgtgaca ccacgatgcc tgtagcaatg ccaacaacgt tgcgcaaact attaactggc 1200
   gaactactta ctctagcttc ccggcaacaa ttaatagact ggatggaggc ggataaagtt 1260
   gcaggaccac ttctgcgctc ggcccttccg gctggctggt ttattgctga taaatctgga 1320
   gccggtgagc gtgggtctcg cggtatcatt gcagcactgg ggccagatgg taagccctcc 1380
   cgtatcgtag ttatctacac gacggggagt caggcaacta tggatgaacg aaatagacag 1440
   atcgctgaga taggtgcctc actgattaag cattggtaac tgtcagacca agtttactca 1500
   tatatacttt agattgattt aaaacttcat ttttaattta aaaggatcta ggtgaagatc 1560
   ctttttgata atctcatgac caaaatccct taacgtgagt tttcgttcca ctgagcgtca 1620
   gaccccgtag aaaagatcaa aggatcttct tgagatcctt tttttctgcg cgtaatctgc 1680
   tgcttgcaaa caaaaaaacc accgctacca gcggtggttt gtttgccgga tcaagagcta 1740
   ccaactcttt ttccgaaggt aactggcttc agcagagcgc agataccaaa tactgtcctt 1800
   ctagtgtagc cgtagttagg ccaccacttc aagaactctg tagcaccgcc tacatacctc 1860
   gctctgctaa tcctgttacc agtggctgct gccagtggcg ataagtcgtg tcttaccggg 1920
   ttggtctcaa gacgatagtt accggataag gcgcagcggt cgggctgaac ggggggttcg 1980
   tgcacacagc ccagcttgga gcgaacgacc tacaccgaac tgagatacct acagcgtgag 2040
   ctatgagaaa gcgccacgct tcccgaaggg agaaaggcgg acaggtatcc ggtaagcggc 2100
   agggtcggta caggagagcg cacgagggag cttccagggg gaaacgcctg gtatctttat 2160
   agtcctgtcg ggtttcgcca cctctgactt gagcgtcgat ttttgtgatg ctcgtcaggg 2220
   gggcggagcc tatcgaaaaa cgccagcaac gcggcctttt tacggttcct ggccttttgc 2280
   tggccttttg ctcacatgtt ctttcctgcg ttatcccctg attctgtgga taaccgtatt 2340
   accgcctttg agtgagctga taccgctcgc cgcagccgaa cgaccgagcg cagcgagtca 2400
   gtgagcgagg aagcggaaga gcgcccaata cgcaaaccgc ctctccccgc gcgttggccg 2460
   attcattaat gcagctggca cgacaggttt cccgactgga aagcgggcag tgagcgcaac 2520
   gcaattaatg tgagttagct cactcattag gcaccccagg ctttacactt tatgcttccg 2580
   gctcgtatgt tgtgtggaat tgtgagcgga taacaatttc acacaggaaa cagctatgac 2640
   catgattacg ccaagctatt taggtgacac tatagaatac tcaagcttgc atgcctgcaa 2700
   tgaggagtat ggatccgact gcagtcggat ccataatcct cgcggccgcg atatcgaatt 2760
   cgccc 2765
<210> 18
   <211> 82
   <212> DNA
   <213> ARTIFICIAL
<220>
   <223> diPET, from pGIS4a2 - sense
<220>
   <221> misc_feature
   <222> (1)..(34)
   <223> n is a, c, g, or t
<220>
   <221> misc_feature
   <222> (47)..(80)
   <223> n is a, c, g, or t
<400> 18
   nnnnnnnnnn nnnnnnnnnn nnnnnnnnnn nnnngtcgga tccgacnnnn nnnnnnnnnn 60
   nnnnnnnnnn nnnnnnnnnn aa 82
<210> 19
   <211> 82
   <212> DNA
   <213> ARTIFICIAL
<220>
   <223> diPET, from pGIS4a2 - antisense
<220>
   <221> misc_feature
   <222> (3)..(36)
   <223> n is a, c, g, or t
<220>
   <221> misc_feature
   <222> (49)..(82)
   <223> n is a, c, g, or t
<400> 19
   aannnnnnnn nnnnnnnnnn nnnnnnnnnn nnnnnncagc ctaggctgnn nnnnnnnnnn 60
   nnnnnnnnnn nnnnnnnnnn nn 82
<210> 20
   <211> 90
   <212> DNA
   <213> ARTIFICIAL
<220>
   <223> diPET, from pGIS3h - sense
<220>
   <221> misc_feature
   <222> (3)..(38)
   <223> n is a, c, g, or t
<220>
   <221> misc_feature
   <222> (51)..(86)
   <223> n is a, c, g, or t
<400> 20
   acnnnnnnnn nnnnnnnnnn nnnnnnnnnn nnnnnnnngt cggatccgac nnnnnnnnnn      60
   nnnnnnnnnn nnnnnnnnnn nnnnnngtcg                                        90
<210> 21
   <211> 90
   <212> DNA
   <213> ARTIFICIAL
<220>
   <223> diPET, from pGIS3h - antisense
<220>
   <221> misc_feature
   <222> (5)..(40)
   <223> n is a, c, g, or t
<220>
   <221> misc_feature
   <222> (53)..(88)
   <223> n is a, c, g, or t
<400> 21
   gctgnnnnnn nnnnnnnnnn nnnnnnnnnn nnnnnnnnnn cagcctaggc tgnnnnnnnn      60
   nnnnnnnnnn nnnnnnnnnn nnnnnnnnca                                        90